# EUROPÄISCHE PATENTSCHRIFT

(11) **EP 0 839 126 B1**
(45) Veröffentlichungstag und Bekanntmachung des Hinweises auf die Patenterteilung: **23.05.2001**
(21) Anmeldenummer: 96924873.1
(22) Anmeldetag: 04.07.1996
(51) Int. Cl.: C07C 209/34, C07C 249/10, C07C 209/26, C07C 211/03, C07C 251/38, C07C 291/02, C07C 49/04, C07C 251/08, C08F 8/30, C10L 1/22, C10L 1/18

(54) **VERFAHREN ZUR HERSTELLUNG VON ORGANISCHEN STICKSTOFFVERBINDUNGEN, SPEZIELLE ORGANISCHE STICKSTOFFVERBINDUNGEN UND MISCHUNGEN AUS SOLCHEN VERBINDUNGEN SOWIE DEREN VERWENDUNG ALS KRAFT- UND SCHMIERSTOFFADDITIVE**
PROCESS FOR PRODUCING ORGANIC NITROGEN COMPOUNDS, SPECIAL ORGANIC NITROGEN COMPOUNDS AND MIXTURES OF SUCH COMPOUNDS AND THEIR USE AS FUEL AND LUBRICANT ADDITIVES
PROCEDE DE PRODUCTION DE COMPOSES AZOTES ORGANIQUES, DE COMPOSES AZOTES ORGANIQUES SPECIAUX ET DE MELANGES CES COMPOSES, AINSI QUE LEUR UTILISATION COMME ADDITIFS POUR CARBURANTS ET LUBRIFIANTS

(30) Priorität: 17.07.1995 DE 19525938
(43) Veröffentlichungstag der Anmeldung: 06.05.1998
(73) Patentinhaber: BASF AKTIENGESELLSCHAFT, 67056 Ludwigshafen (DE)
(72) Erfinder: KROPP, Rudolf, D-67117 Limburgerhof (DE); SIEGEL, Wolfgang, D-67117 Limburgerhof (DE); BREITSCHEIDEL, Boris, D-67117 Limburgerhof (DE); HARDER, Wolfgang, D-69469 Weinheim (DE); SCHWAHN, Harald, D-69168 Wiesloch (DE); REIF, Wolfgang, D-67227 Frankenthal (DE)
(86) Internationale Anmeldenummer: EP9602928
(87) Internationale Veröffentlichungsnummer: WO9703946

(56) Entgegenhaltungen:
- EP-A- 0 384 086
- EP-A- 0 606 976
- WO-A-96/03367
- DE-A- 1 545 487
- FR-A- 2 687 159
- GB-A- 1 172 818
- DATABASE CROSSFIRE beilstein Informationssysteme GmbH XP002017950 & CENTRALBLATT, 1913, Seite 1376
- DATABASE CROSSFIRE beilstein Informationssysteme GmbH XP002017951 & J. CHEM. SOC, 1955, Seite 1547, 1551
- DATABASE CROSSFIRE Beilstein Informationssysteme GmbH XP002017952 & POL. J. CHEM., Bd. 60, Nr. 4-6, 1986, Seiten 625-30,
- DATABASE CROSSFIRE Beilstein Informationssysteme GmbH XP002017953 & J. SCI., Bd. 30, 1926, Seite 206
- DATABASE CROSSFIRE Beilstein Informationssysteme GmbH XP002017954 & HETEROCYCLES, Bd. 36, Nr. 8, 1993, Seiten 1823-36,
- DATABASE CROSSFIRE Beilstein Informationssysteme GmbH XP002017955 & HEL. CHIM. ACTA, Bd. 11, 1928, Seite 694, 695
- DATABASE CROSSFIRE Beilstein Informationssysteme GmbH XP002017956 & J. CHEM. SOC., Bd. 85, 1904, Seite 832
- DATABASE CROSSFIRE Beilstein Informationssysteme GmbH XP002017957 & C. R. HEBD. SEANCES ACAD. SCI., Bd. 224, 1947, Seite 1116, 1117

## Beschreibung

Die vorliegende Erfindung betrifft ein Verfahren zur Herstellung von organischen Stickstoffverbindungen, insbesondere von Aminoalkanen, Alkyloximen, Alkylnitronen oder Mischungen hieraus, aus Nitrogruppen enthaltenden Umsetzungsprodukten von Olefinpolymerisaten mit Stickoxiden. Weiterhin betrifft die Erfindung Mischungen aus solchen speziellen organischen Stickstoffverbindungen.

Vergaser und Einlaßsystem von Ottomotoren, aber auch Einspritzsysteme für die Kraftstoffdosierung in Otto- und Dieselmotoren, werden durch Verunreinigungen belastet, die durch Staubteilchen aus der Luft, unverbrannte Kohlenwasserstoffreste aus dem Brennraum und die in den Vergaser geleiteten Kurbelwellengehäuseentlüftungsgase verursacht werden.

Die Rückstände verschieben das Luft-Kraftstoffverhältnis im Leerlauf und im unteren Teillastbereich, so daß das Gemisch fetter, die Verbrennung unvollständiger und wiederum die Anteile unverbrannter oder teilverbrannter Kohlenwasserstoffe im Abgas größer werden und der Benzinverbrauch steigt.

Es ist bekannt, daß zur Vermeidung dieser Nachteile Kraftstoffadditive zur Reinhaltung von Ventilen und Vergaser bzw. Einspritzsystemen verwendet werden (M. Rossenbeck in Katalysatoren, Tenside, Mineralöladditive, Hrsg. J. Falbe, U. Hasserodt, S. 233 f., G. Thieme Verlag, Stuttgart, 1978).

Je nach Wirkungsweise, aber auch nach dem bevorzugten Wirkort solcher Detergents-Additive unterscheidet man heute zwei Generationen derartiger Hilfsmittel.

Die erste Additiv-Generation konnte nur die Bildung von Ablagerungen im Ansaugsystem verhindern, nicht aber bereits vorhandene Ablagerungen wieder entfernen, wohingegen die Additive der zweiten Generation beides bewirken können ("keep-clean-" und "clean-up-Effekt") und zwar aufgrund ihrer hervorragenden ThermoStabilität, insbesondere auch an Zonen höherer Temperaturen, nämlich an den Einlaßventilen.

Das molekulare Bauprinzip von Kraftstoff-Detergentien kann verallgemeinernd angegeben werden als Verknüpfung polarer Strukturen mit meist höhermolekularen, unpolaren oder lipophilen Resten.

Vertreter der zweiten Additiv-Generation sind oft Produkte auf der Basis von Polyisobutenen im unpolaren Molekülteil. Hier wieder sind Additive von Polyisobutylamin-Typ besonders hervorzuheben.

Umsetzungsprodukte von höheren Olefinen wie Polyisobutenen oder Oligopropenen mit Stickoxiden und deren Verwendung als Additive für Mineralölprodukte sind aus den deutschen Patentanmeldungen P 44 25 834.8 (1) und P 44 25 835.6 (2) bekannt. Dort werden teilweise auch aus solchen Nitrogruppen enthaltenden Umsetzungsprodukten ableitbare Aminoalkane und Wege zu deren Herstellung beschrieben.

In der US-A 3 681 463 (3) werden die Herstellung öllöslicher Aminoalkylalkohole auf Basis von Polypropen und Polybuten mit überwiegend nicht α-ständigen Doppelbindungen durch Nitrierung der zugrunde liegenden Olefine mit Stickstofftetroxid zu den entsprechenden Nitronitratestern und die Reduktion dieser Nitronitratester mit Wasserstoff in Gegenwart eines metallischen Hydrierungskatalysators beschrieben. Die so erhaltenen Aminoalkylalkohole eignen sich als Additive für Mineralölprodukte.

Aus der FR-A 2 687 159 (4) ist bekannt, daß man durch Umsetzung von Polybutenen mit wäßriger Salpetersäure und anschließende Behandlung mit Basen unter Ausbildung von Polybutenderivaten mit reaktiven Carbonylgruppen und weitere Umsetzung dieser Polybutenderivate mit Aminen und anschließende Hydrierung stickstoffhaltige Polybutene erhalten kann, die sich als Kraft- oder Schmierstoffadditive eignen.

Die Wirkung der aus dem Stand der Technik bekannten stickstoffhaltigen Polyolefinderivate als Additive für Mineralölprodukte ist noch verbesserungsbedürftig. Daher lag der vorliegenden Erfindung die Aufgabe zugrunde, Kraft- und Schmierstoffadditive mit verbesserter Wirkung bereitzustellen. Dabei sollten solche Additive außerdem durch einfache und wirtschaftliche Verfahren in guten Ausbeuten und hohen Reinheiten herstellbar sein.

Demgemäß wurde ein Verfahren zur Herstellung von organischen Stickstoffverbindungen, insbesondere von Aminoalkanen, Alkyloximen, Alkylnitronen oder Mischungen hieraus, welche nur eine stickstoffunktionelle Gruppierung und keine alkoholischen Hydroxylgruppen im Molekül tragen, aus Nitrogruppen enthaltenden Umsetzungsprodukten von Polymerisaten von C₂- bis C₆-Olefinen mit einem mittleren Polymerisationsgrad P = 5 bis 100 mit Stickoxiden oder Gemischen aus Stickoxiden und Sauerstoff gefunden, welches dadurch gekennzeichnet ist, daß man die Nitrogruppen enthaltenden Umsetzungsprodukte direkt im Anschluß an ihre Bildung aus den Olefinpolymerisaten und den Stickoxiden hydriert.

Die genannten Aminoalkane, Alkyloxime und/oder Alkylnitrone können jedoch nicht nur durch direkte Hydrierung der Nitrogruppen enthaltenden Umsetzungsprodukte hergestellt werden. Im Sinne der vorliegenden Erfindung kann man auch derart vorgehen, daß man die Nitrogruppen enthaltenden Umsetzungsprodukte im Anschluß an ihre Bildung durch Eliminierung mit Basen in Nitrogruppen enthaltende Alkene umwandelt und dieser danach hydriert, wobei resultierende Aminoalkane immer in Form von Mischungen von Verbindungen mit unterschiedlicher Anzahl von C-Atomen anfallen.

Typische Strukturelemente für derartige Nitrogruppen enthaltende Alkene sind die folgenden: 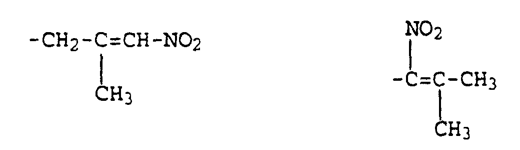 welche beispielsweise ursprünglich aus einem Polyisobuten mit endständiger Doppelbindung (links) oder aus einem Polyisobuten mit β-ständiger Doppelbindung (rechts) entstanden sind. Auch Hydroxylgruppen enthaltende Verbindungen können Nebenprodukte solcher vorgeschobenen Eliminierungsreaktionen sein. In der Regel liegt als Produkt der Eliminierungsreaktion eine Mischung verschiedener Species vor, in der Verbindungen mit dem linken obigen Strukturelement die Hauptkomponente bilden und Verbindungen mit dem rechten obigen Strukturelement nur in geringen Mengen oder gar nicht vorkommen.

Derartige Eliminierungsreaktionen werden unter den hierfür übliche Bedingungen durchgeführt. Als Basen setzt man beispielsweise Alkalimetallhydroxide wie NaOH oder KOH, Alkalimetallalkoholate wie Natriummethanolat, Natriumethanolat, Natriumisopropylat oder Kalium-tert.-butylat oder insbesondere Alkalimetallcarbonate oder -hydrogencarbonate wie Natrium- oder Kaliumcarbonat oder Natrium- oder Kaliumhydrogencarbonat ein. Weiterhin eignen sich unter speziellen Bedingungen hier auch Ammoniak, Amine allgemein, Alkalimetallfluoride oder heterogene Basensysteme wie basische Ionentauscher als Basen für die Eliminierungsreaktionen.

In einer weiteren Ausführungsform der vorliegenden Erfindung kann man die Nitrogruppen enthaltenden Umsetzungsprodukte vor der Hydrierung in Verbindungen mit reaktiven Carbonylgruppen überführen und diese mit Ammoniak oder primären Aminen zu Iminen umsetzen. Hierbei kann die Zwischenstufe der reaktiven Carbonylverbindung isoliert oder gleich in situ mit der NH₂-Gruppe des zugesetzten Amins zum entsprechenden Imin umgesetzt werden. Dabei können die resultierenden organischen Stickstoffverbindungen, herrührend von den eingesetzten primären Aminen, auch mehrere stickstoffunktionelle Gruppen und alkoholische Hydroxylgruppen im Molekül tragen.

Eine typische derartige Reaktionsfolge kann folgendermaßen dargestellt werden:

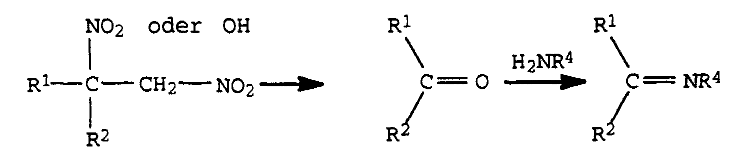

Als primäre Amine für diese Iminbildung eignen sich beispielsweise C₁- bis C₃₀-Alkylamine oder C₃- bis C₃₀-Alkenylamine, bei denen die Alk(en)ylreste durch nicht benachbarte Sauerstoffatome oder durch NR²-Gruppen, wobei R² für Wasserstoff oder für C₁- bis C₃-Alkyl steht, unterbrochen sein oder Hydroxylgruppen tragen können, C₅- bis C₈-Cycloalkylamine, C₇- bis C₁₈-Aralkylamine, gegebenenfalls substituierte C₆- bis C₁₄-Arylamine, Diamine oder Polyamine der allgemeinen Formel H₂N(̵A-NR⁷)̵ₘ-R⁸, Alkanolamine der allgemeinen Formel H₂N-A-OH, Etheramine, Oligo- und Polyetheramine der allgemeinen Formel H₂N(̵A-O)ₘ-R⁷ oder Oligo- und Polyetheralkanolamine der allgemeinen Formel H₂N(̵A-O)ₘ-A-OH, in denen A für C₂-bis C₁₀-Alkylen, C₅- bis C₁₈-Cycloalkylen oder Phenylen, R⁷ und R⁸ für Wasserstoff oder C₁- bis C₈-Alkyl und m für eine Zahl von 1 bis 8 stehen. Typische Einzelbeispiele für solche primären Amine sind Methylamin, Ethylamin, n-Propylamin, Isopropylamin, n-Butylamin, Isobutylamin, tert.-Butylamin, 2-Ethylhexylamin, Stearylamin, Oleylamin, Allylamin, Cyclohexylamin, Benzylamin, Anilin, Toluidine, 1,2-Diethylendiamin, 1,3-Dipropylendiamin, 3-(N,N-Dimethylamino)propylamin, Diethylentriamin, Triethylentetramin, Tetraethylenpentamin, Ethanolamin und Hexamethylendiamin.

Die bezeichneten primären Amine werden entweder direkt mit den Nitrogruppen enthaltenden Umsetzungsprodukten oder mit den daraus beispielsweise durch Umsetzung mit Basen ableitbaren reaktive Carbonylgruppen enthaltenden Verbindungen in der Regel bei Temperaturen von 5 bis 150°C, insbesondere 20 bis 100°C, meist in einem üblichen inerten organischen oder anorganischen Lösungsmittel und üblicherweise bei Normaldruck umgesetzt.

Die Hydrierung der Nitrogruppen enthaltenden Umsetzungsprodukte direkt oder deren Folgeprodukte wird vorzugsweise als katalytische Hydrierung mit Wasserstoff in Gegenwart von Hydrierungskatalysatoren, als Transferhydrierung mit reduzierend wirkenden organischen oder anorganischen Molekülverbindungen, als Reduktion mit unedlen Metallen oder als Reduktion mit salzartigen komplexen Hydriden oder salzartigen niedervalenten Schwefelverbindungen durchgeführt.

Die Hydrierung kann drucklos oder unter Druck, sowohl kontinuierlich als auch diskontinuierlich in den hierfür üblichen Reaktortypen wie z.B. Rührkesseln, Rührkesselkaskaden, Rohrreaktoren, Blasensäulen und Mischformen dieser Reaktortypen durchgeführt werden.

Die Hydrierung wird im allgemeinen in einem inerten Lösungs- oder Verdünnungsmittel oder in einer Mischung solcher Mittel durchgeführt. Hierfür eignen sich beispielsweise Kohlenwasserstoffe wie n-Hexan, Isooctan, n-Alkan-Gemisch (z.B. C₉-C₁₃), Cyclohexan, Toluol oder Tetralin, Ether wie Diethylether, tert.-Butyl-methylether oder Tetrahydrofuran, Alkohole wie Methanol, Isopropanol oder 2-Ethylhexanol, Ester wie Essigsäureethyl- oder n-butylester oder Amide wie Dimethylformamid oder N-Methylpyrrolidon. Wenn die Reaktionsprodukte als Kraftstoffadditive Verwendung finden sollen, arbeitet man zweckmäßigerweise im gleichen Lösungsmittel, in dem es später dem Kraftstoff zugemischt wird. Im allgemeinen betragen die Lösungsmittelmengen 50 bis 90 Gew.-% des Gesamtansatzes. Es kann aber auch ohne Lösungsmittel gearbeitet werden.

Die Temperatur bei der Hydrierung kann normalerweise im Bereich von 20°C bis 250°C variiert werden. Sie ist abhängig vom angewandten Reduktionssystem. Bei katalytischen Hydrierungen liegt ein bevorzugter Temperaturbereich von 150°C bis 220°C.

Der Wasserstoffdruck bei der katalytischen Hydrierung kann in der Regel von 1 bar bis 300 bar eingestellt werden, bevorzugt beträgt er 100 bar bis 200 bar.

Die bei der katalytischen Hydrierung mit Wasserstoff zur Anwendung kommenden Hydrierungskatalysatoren sind beispielsweise Edelmetallkatalysatoren wie Platin, Palladium, Ruthenium, Rhodium, Osmium oder Iridium, Raney-Katalysatoren wie Nickel, Kobalt, Eisen oder Kupfer, Mischkatalysatoren, die beispielsweise Nickel, Zirkonium, Kupfer und Molybdän oder Kupfer, Chrom, Zink und Barium enthalten, oder oxidische und sulfidische Katalysatoren. Die Katalysatoren können in reiner Form in homogener Lösung oder heterogen in Suspension oder als Trägerkatalysatoren eingesetzt werden. Als Träger finden beispielsweise Kohle, Aluminiumoxid, Zirkonoxid, Siliziumdioxid oder Magnesiumoxid Anwendung.

In den diskontinuierlichen katalytischen Hydrierungen beträgt die Menge des Hydrierungskatalysators meist 0,01 bis 50 Gew.-%, vorzugsweise 1 bis 25 Gew.-%, bezogen auf die eingesetzten Nitrogruppen enthaltenden Umsetzungsprodukte.

Reduzierend wirkende organische oder anorganische Molekülverbindungen bei Transferhydrierung sind z.B. Ameisensäure oder Hydrazin.

Für die Hydrierung geeignete reduzierende unedle Metalle sind z.B. Eisen, Zink oder Zinn.

Bei der Reduktion mit salzartigen komplexen Hydriden können insbesondere Lithiumaluminiumhydrid, Diisobutylaluminiumhydrid, Natriumborhydrid oder Tributylzinnhydrid verwendet werden. Als salzartige niedervalente Schwefelverbindung kommt für die Reduktion hier z.B. Alkalimetalldithionit in Betracht.

Die Hydrierung kann sowohl in Abwesenheit als auch in Anwesenheit von Ammoniak oder primären, sekundären oder tertiären Aminen, Diaminen, Polyaminen, Alkanolaminen, Etheraminen, Polyetheraminen oder Polyetheralkanolaminen (R⁴NH₂, R⁴R⁵NH bzw. (R⁴)₃N) durchgeführt werden. Die ein bis drei organischen Reste dieser Amine stehen dabei jeweils unabhängig voneinander für die nachfolgend definierten Reste R⁴ bzw. R⁵. Der Zusatz eines derartigen primären oder sekundären Amins bewirkt insbesondere den Einbau des zugesetzten Amins in das Produkt. Dabei können die resultierenden organischen Stickstoffverbindungen, herrührend von den eingesetzten primären oder sekundären Aminen, auch mehrere stickstoffunktionelle Gruppen und alkoholische Hydroxylgruppen im Molekül tragen.

Die Menge von zugesetztem Ammoniak oder Aminen kann bis 200 Gew.-%, vorzugsweise 10 bis 100 Gew.-%, bezogen auf die eingesetzten Nitrogruppen enthaltenden Umsetzungsprodukte, betragen. Neben Ammoniak eignen sich hier beispielsweise folgende Amine:

Methylamin, tert.-Butylamin, 2-Ethylhexylamin, 1,2-Ethylendiamin, Hexamethylendiamin, 3-(N,N-Dimethylamino)propylamin, Benzylamin, Anilin, p-Methoxyanilin, m-Phenylendiamin, Dipropylentriamin, 1,4-Diaminocyclohexan, 4,4'-Diaminodicyclohexylmethan, Dimethylamin, Diethanolamin, Di(tridecyl)amin, Pyrrolidin, Morpholin, Piperazin, Triethylamin, N,N-Dimethylanilin oder Pyridin.

Die Hydrierung wird vorteilhafterweise unter neutralen oder basischen Reaktionsbedingungen durchgeführt.

Für das erfindungsgemäße Verfahren sind katalytische Hydrierungen mit Wasserstoff in Gegenwart von Hydrierungskatalysatoren besonders vorteilhaft, da bei Verwendung anderer Reduktionsmethoden manchmal Reste von Metallen, Metallsalzen, Schwefelverbindungen oder ähnliche Verunreinigungen im Reduktionsprodukt verbleiben, die bei Verwendung als Kraft- und Schmierstoffadditiv die Wirkung des Abgaskatalysators von Ottomotor-angetriebenen Kraftfahrzeugen beeinträchtigen können.

Die Hydrierung kann sowohl kontinuierlich als auch diskontinuierlich in hierfür üblichen Apparaturen durchgeführt werden.

Als C₂- bis C₆-Olefine für die als Ausgangsmaterial eingesetzten Olefinpolymerisate können Ethylen, Propen, 1-Buten, 2-Buten, Isobuten, 1,3-Butadien, 1-Penten, 2-Penten, 2-Methyl-1-buten, 2-Methyl-2-buten, 1,3-Pentadien, 1-Hexen, 2-Hexen, 3-Hexen, 2-Methyl-1-penten, 2-Methyl-2-penten, 2-Methyl-3-penten, 2-Methyl-4-penten, 3-Methyl-1-penten, 3-Methyl-2-penten, 2-Ethyl-1-buten, 3,3-Dimethyl-l-buten, 1,3-Hexadien, 2,4-Hexadien, 1,5-Hexadien oder 1,3,5-Hexatrien verwendet werden. Es können auch Mischungen der genannten Olefine eingesetzt werden. Bevorzugt werden hiervon Propen, 1-Buten, 2-Buten, Isobuten, 1,3-Butadien oder Mischungen hieraus. Ein typisches Beispiel für ein derartiges Olefinpolymerisat ist Polypropylen.

Für das erfindungsgemäße Verfahren eignen sich als einsetzbare Nitrogruppen enthaltende Umsetzungsprodukte insbesondere solche von Polymerisaten von Isobuten mit einem mittleren Polymerisationsgrad P = 5 bis 100, bei denen bis zu 50 Gew.-%, vorzugsweise bis zu 30 Gew.-% des Isobutens durch andere C₂- bis C₆-Olefine, insbesondere durch Propen, 1-Buten, 2-Buten oder 1,3-Butadien oder eine Mischung hieraus, als Comonomere ersetzt sein können, mit Stickoxiden oder Gemischen aus Stickoxiden und Sauerstoff.

Die Polymerisation der genannten C₂- bis C₆-Olefine erfolgt in der Regel nach üblichen Methoden. Die Polymerisate weisen aufgrund von Kettenabbruchreaktionen endständige (α-ständige), β-ständige und innerständige Doppelbindungen auf, wobei die β-ständigen und insbesondere die endständigen Doppelbindungen die bevorzugten Reaktionszentren für die Umsetzung mit den Stickoxiden darstellen.

Der mittlere Polymerisationsgrad P liegt für die vorgenannten Ausführungsformen bei 5 bis 100, vorzugsweise 8 bis 80, insbesondere 10 bis 60, vor allem 15 bis 40. Wie stets bei derartigen Polymerisationen erhält man Polymere mit einem bestimmten Polymerisationsgradspektrum. Die Streuung ist jedoch im Hinblick auf die Eigenschaften der Umsetzungsprodukte mit Stickoxiden bzw. Stickoxid-Sauerstoff-Gemischen ohne erkennbaren Einfluß, so daß es nur auf den mittleren Polymerisationsgrad P ankommt, der beispielsweise durch Viskositätsmessungen auch während der Polymerisation laufend ermittelt und gesteuert werden kann.

In Korrelation mit dem mittleren Polymerisationsgrad P weisen die beschriebenen Polyolefine Kohlenstoffzahlen von 10 bis ca. 600, vorzugsweise 24 bis ca. 320, insbesondere 40 bis ca. 240, und mittlere Molekulargewichte (zahlengemittelt) von 140 bis 8400, vorzugsweise 330 bis 4500, insbesondere 560 bis 3400, auf.

Für die Umsetzung zu den beschriebenen Produkten kommen generell als Stickoxide vor allem Stickstoffmonoxid (NO), Stickstoffdioxid (NO₂), Distickstofftrioxid (N₂O₃), Distickstofftetraoxid (N₂O₄), Gemische dieser Stickoxide untereinander sowie Gemische dieser Stickoxide mit Sauerstoff, insbesondere NO mit Sauerstoff und NO₂ mit Sauerstoff, in Betracht. Bei Mitverwendung von Sauerstoff macht dieser im Gemisch mit den Stickoxiden 1 bis 70 Vol.-%, insbesondere 5 bis 50 Vol.-% aus. Das Stickoxid-Sauerstoff-Gemisch kann auch noch Inertgase, z.B. Stickstoff, enthalten; dies tritt beispielsweise auf, wenn man Stickoxid-Luft-Gemische verwendet.

Die Umsetzung zu den beschriebenen Produkten kann drucklos oder unter Druck, diskontinuierlich oder kontinuierlich durchgeführt werden.

Um einen quantitativen Umsatz zu erzielen, werden die Stickoxide im Molverhältnis Polyolefine zu Stickoxid von 1 : 2 bis 1 : 4, vorzugsweise 1 : 2,2 bis 1 : 3,3 zugegeben. Ein größerer Überschuß schadet nicht.

Die Temperatur ist unkritisch. Sie kann im Bereich von -30° bis 150°C variiert werden. Bevorzugt arbeitet man bei -10° bis 100°C, insbesondere bei 25°C bis 80°C.

Die Umsetzung wird in vorteilhafter Weise in einem inerten organischen Lösungsmittel durchgeführt. Dafür eignen sich beispielsweise aliphatische Kohlenwasserstoffe wie Isooctan oder ein n-Alkan-Gemisch (z.B. C₁₀-C₁₃), chlorierte Kohlenwasserstoffe wie Methylenchlorid, Tetrachlorkohlenstoff oder Chlorbenzol, Ether wie Diethylether, Tetrahydrofuran, Dioxan oder tert.-Butylmethylether, Ester wie Essigsäureethylester oder Benzoesäuremethylester, Amide wie Dimethylformamid oder N-Methylpyrrolidon sowie Säuren wie Essigsäure. Im allgemeinen betragen die Lösungsmittelmengen 50 bis 90 Gew.-% des Gesamtansatzes. Es kann aber auch ohne Lösungsmittel gearbeitet werden.

Der Zusatz einer geringen Menge Wasser (etwa 0,2 bis 1 Gew.-%, bezogen auf eingesetztes Polyolefin), um eventuell gebildeten Nitritester zu hydrolysieren, schadet nicht.

Die Aufarbeitung eines Reaktionsansatzes geschieht meist in der Weise, daß entweder kurz im Vakuum auf 40 bis 50°C erhitzt oder mit Wasser gerührt und anschließend eine Phasentrennung vorgenommen wird. Beide Maßnahmen haben das Ziel, Reste von Stickoxiden aus dem Reaktionsgemisch zu entfernen.

In der Regel fallen die beschriebene Nitrogruppen enthaltenden Umsetzungsprodukte, insbesondere wenn NO₂ als Stickoxid eingesetzt oder mitverwendet wurde, in Form einer Mischung verschiedener Nitrogruppen enthaltender Alkane an.

Für das erfindungsgemäße Verfahren eignen sich als einsetzbare Nitrogruppen enthaltende Umsetzungsprodukte weiterhin insbesondere solche von Polymerisaten von C₂- bis C₆-Olefinen mit einem mittleren Polymerisationsgrad P = 5 bis 100 und Stickoxiden oder Gemischen aus Stickoxiden und Sauerstoff, die in Form einer Mischung verschiedener Nitrogruppen enthaltender Alkane vorliegen und als Hauptkomponenten die Verbindung der allgemeinen Formel I und II 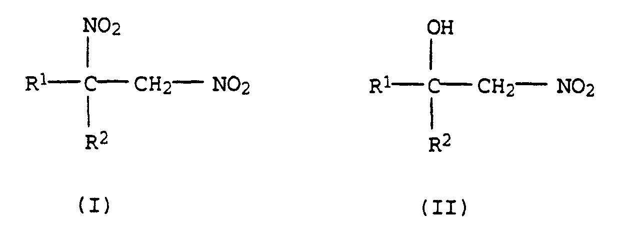 in denen
- R¹: einen langkettigen linearen oder verzweigten Alkylrest mit 8 bis 600, vorzugsweise 20 bis 450, insbesondere 40 bis 300 C-Atomen bezeichnet und
- R²: für Wasserstoff oder C₁- bis C₃-Alkyl steht,
enthalten. Die Verbindungen I und II leiten sich von Olefinpolymerisaten mit endständigen Doppelbindungen ab.

Für das erfindungsgemäße Verfahren eignen sich als einsetzbare Nitrogruppen enthaltende Umsetzungsprodukte weiterhin insbesondere solche von Polymerisaten von C₂- bis C₆-Olefinen mit einem mittleren Polymerisationsgrad P = 5 bis 100 und einem hohen Anteil an β-ständigen und einem geringen Anteil an endständigen Doppelbindungen mit Stickoxiden oder Gemischen aus Stickoxiden und Sauerstoff, die in Form einer Mischung verschiedener Nitrogruppen enthaltender Alkane vorliegen und als Hauptkomponenten die Verbindungen der allgemeinen Formel III und IV 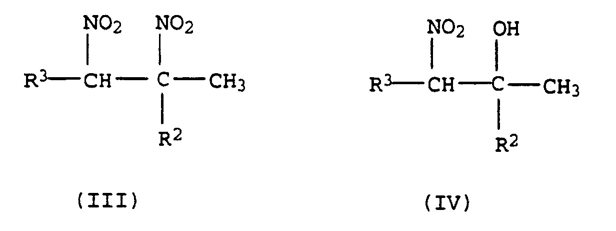 in denen
- R³: einen langkettigen linearen oder verzweigten Alkylrest mit 8 bis 600, vorzugsweise 20 bis 450, insbesondere 40 bis 300 C-Atomen bezeichnet und
- R²: für Wasserstoff oder C₁- bis C₃-Alkyl steht,
enthalten. Dabei stellt der Rest R³ einen um ein Kohlenstoffatom oder eine CH₂-Gruppe verkürzten Rest R¹ dar.

Für das erfindungsgemäße Verfahren eignen sich als einsetzbare Nitrogruppen enthaltende Umsetzungsprodukte weiterhin insbesondere solche von Polyisobutenen mit einem mittleren Polymerisationsgrad P = 10 bis 100 mit einem Anteil E = 60 bis 90 % an Doppelbindungen, die mit Maleinsäureanhydrid umsetzbar sind, woi bei E = 100 % dem rechnerisch-theoretischen Wert für den Fall entspräche, daß jedes Molekül des Polyisobutens eine derartige reaktive Doppelbindung hätte, mit Stickoxiden oder Gemischen aus Stickoxiden und Sauerstoff.

Der mittlere Polymerisationsgrad P liegt für die beschriebenen hochreaktiven Polyisobutene bei 10 bis 100, vorzugsweise bei 15 bis 40. In Korrelation mit dem mittleren Polymerisationsgrad P weisen die beschriebenen hochreaktiven Polyisobutene Kohlenstoffzahlen von 36 bis 400, vorzugsweise 54 bis 160 und mittlere Molekulargewichte (zahlengemittelt) von 500 bis 5600, vorzugsweise 750 bis 2250 auf.

Unter dem Begriff Polyisobutene sind als Ausgangsmaterialien für die vorliegende Erfindung nicht nur die Homopolymerisate des Isobutens, sondern auch dessen Copolymerisate mit mindestens 80 % Isobutenanteil zu verstehen. Als Comonomere kommen in erster Linie die übrigen olefinisch ungesättigten C₄-Kohlenwasserstoffe in Betracht, so daß man, was von besonderer technischer Bedeutung ist, unmittelbar von den sogenannten C₄-Schnitten ausgehen kann. Diese erhalten neben 12 bis 14 % Butanen, 40 bis 55 % Butenen und bis zu 1 % Butadien zwar nur 35 bis 45 % Isobuten, jedoch bedingt die weitgehend selektive Polymerisierbarkeit des Isobutens, daß die übrigen Monomeren unter den Polymerisationsbedingungen nur zu etwa 2 bis 20 % in das Polymere eingebaut werden. Die Monomeren, die nicht reagiert haben, können für andere Zwecke verwendet werden. Als weitere Comonomere kommen noch C₃-Monomere wie Propen sowie Ethylen oder Mischung hieraus oder mit C₄-Monomeren in Betracht.

Nach diesem Verfahren erhält man Isobutene mit einem Anteil E an Doppelbindungen, die sich mit Maleinsäureanhydrid umsetzen lassen, von 60 bis 90 Prozent, in vielen Fällen von 75 bis 90 Prozent. Der rechnerisch-theoretische Wert von E = 100 % würde hiernach bedeuten, daß jedes Polyisobutenmolekül eine derart reaktionsfähige Doppelbindung enthielte. E ist in einfacher Weise und am zuverlässigsten unmittelbar aus der Säurezahl des Polyisobuten/Maleinsäureanhydrid-Adduktes zu ermitteln.

Bezüglich der einzusetzenden Stickoxide und der Umsetzungsbedingungen gilt für die beschriebenen hochreaktiven Polyisobutene gleichermaßen das oben für die Umsetzung von C₂- bis C₆-Olefinpolymerisaten Gesagte.

Für das erfindungsgemäße Verfahren eignen sich als einsetzbare Nitrogruppen enthaltende Umsetzungsprodukte weiterhin insbesondere solche von Polyisobutenen mit einem mittleren Polymerisationsgrad P = 10 bis 100 mit einem Anteil E = 60 bis 90 % an Doppelbindungen, die mit Maleinsäureanhydrid umsetzbar sind, wobei E = 100 % dem rechnerisch-theoretischen Wert für den Fall entspräche, daß jedes Molekül des Polyisobutens eine derartige reaktive Doppelbindung hätte, mit Stickoxiden oder Gemischen aus Stickoxiden und Sauerstoff, die in Form einer Mischung verschiedener Nitrogruppen enthaltender Alkane vorliegen und als Hauptkomponenten die Verbindungen der allgemeinen Formel V bis VIII 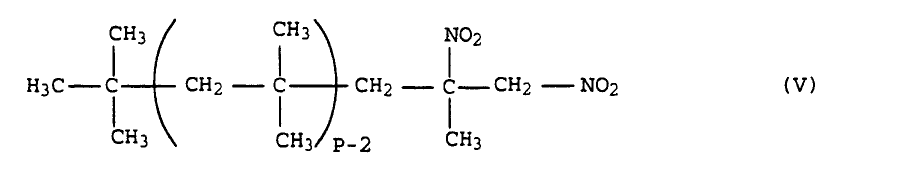 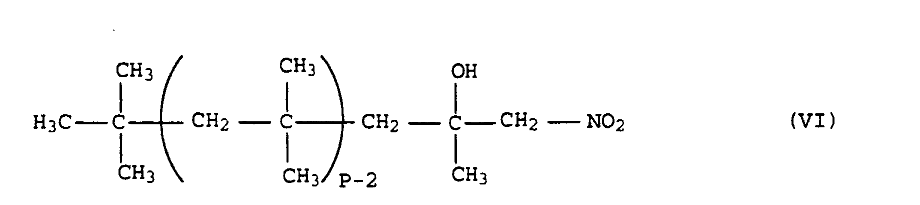 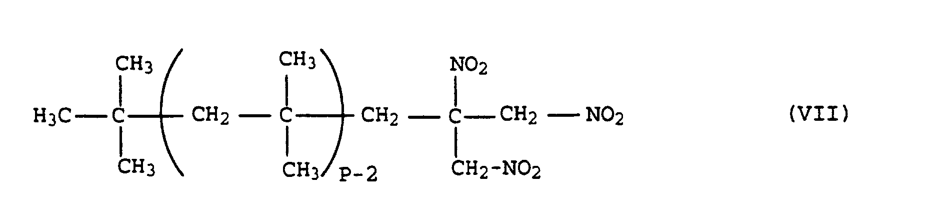 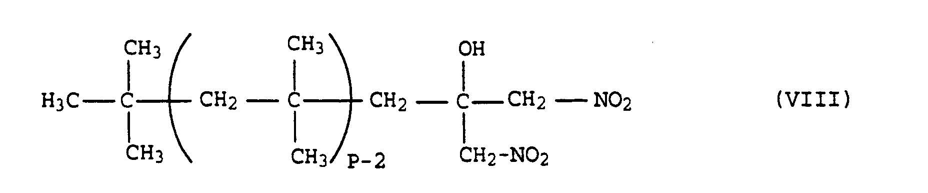 enthalten.

Überraschenderweise werden beim erfindungsgemäßen Verfahren die Produkte, die sich direkt durch Reduktion der Nitrogruppen ableiten, wie z.B. das entsprechende Diamin aus der Dinitroverbindung I, wenn überhaupt, dann nur in untergeordneter Menge gebildet. Je nach den angewandten Reaktionsbedingungen werden Aminoalkane, Alkyloxime und/oder Alkylnitrone der Strukturen IX bis XI oder XII bis XIV als Hauptprodukte erhalten: 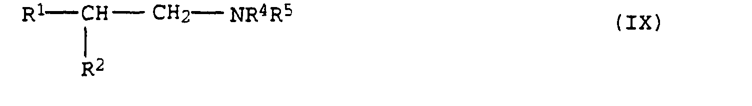 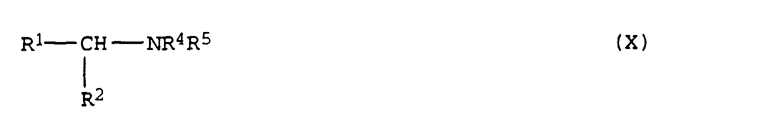 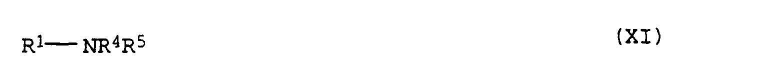 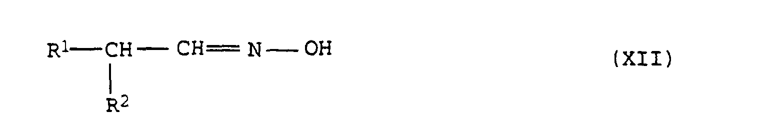 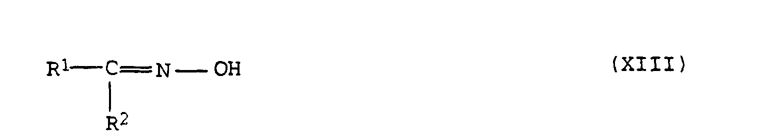 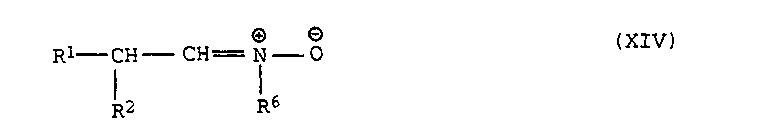 wobei
- R¹: einen langkettigen linearen oder verzweigten Alkylrest mit 8 bis 600, vorzugsweise 20 bis 450, insbesondere 40 bis 300 C-Atomen bezeichnet,
- R²: für Wasserstoff oder C₁- bis C₃-Alkyl steht,
- R⁴: und R⁵ unabhängig voneinander Wasserstoff, C₁- bis C₃₀-Alkyl oder C₃- bis C₃₀-Alkenyl, welches durch nicht benachbarte Sauerstoffatome oder durch NR²-Gruppen unterbrochen sein oder Hydroxylgruppen tragen kann, C₅- bis C₈-Cycloalkyl, C₇- bis C₁₈-Aralkyl, gegebenenfalls substituiertes C₆- bis C₁₄-Aryl, Amin-, Diamin- oder Polyaminreste der Formel (̵A-NR⁷)̵ₘR⁸, Alkanolaminreste der Formel -A-OH, Etheramin-, Oligo- und Polyetheraminreste der Formel (̵A-O)ₘ-R⁷ oder Oligo- und Polyetheralkanolaminreste der Formel (̵A-O)ₘ-A-OH, in denen A für C₂-bis C₁₀-Alkylen, C₅- bis C₁₈-Cycloalkylen oder Phenylen, R⁷ und R⁸ für Wasserstoff oder C₁- bis C₈-Alkyl und m für eine Zahl von 1 bis 8 stehen, wobei die beiden Reste R⁴ und R⁵ auch einen fünf- oder sechsgliedrigen Ring bilden können, bedeuten und
- R⁶: C₁- bis C₃-Alkyl bedeutet.

Die Bedeutung der organischen Reste R⁴ und R⁵ am Aminstickstoff entspricht denen der organischen Reste der primären Amine, die vorn zur Iminbildung verwandt werden.

Die Bedeutung von R⁶ entspricht in der Regel der von R³.

Stehen beide Reste R⁴ und R⁵ nicht für Wasserstoff, stammen die entsprechenden organischen Reste R⁴ bzw. R⁵ im Sinne der vorliegenden Erfindung aus - wie vorne beschrieben - bei der Hydrierung zugesetzten primären oder sekundären Aminen. Bezüglich der Bedeutungen der Variablen R⁴ und R⁵ bei diesen Aminen gilt das oben Ausgeführte.

Demgemäß sind auch Gegenstand der vorliegenden Erfindung derartige Mischungen aus Alkyloximen und Alkylnitronen und gegebenenfalls Aminoalkanen mit den gleichen langkettigen Resten, welche als Hauptkomponenten die Verbindungen der allgemeinen Formel XII bis XIV enthalten.

Die drei angegebenen Hauptkomponenten der erfindungsgemäßen Mischung stehen normalerweise im Gew.-Verhältnis von (1-98):(1-98):(1-98), insbesondere (5-90):(5-90):(5-90). Dieses Verhältnis kann durch die Wahl der Reaktionsparameter eingestellt werden.

Die Strukturen IX bis XIV sowie die Carbonyl- und die Imin-Vorstufen zu diesen Endprodukten können nach üblichen Methoden aus den anfallenden Gemischen isoliert und so in reiner Form erhalten werden. Auch nachträgliche Anreicherungen der betreffenden Strukturen in den anfallenden Gemischen sind möglich. Diese isolierten Einzelstrukturen zeigen zumindest gleichgute anwendungstechnische Eigenschaften. In vielen Fällen ist es auch möglich, die genannten Einzelstrukturen durch geeignete Wahl der Reaktionsparameter in praktisch reiner Form oder zumindest in stark angereicherter Form aus der entsprechenden Umsetzung direkt zu erhalten.

In einer bevorzugten Ausführungsform hat der Rest R¹ bei der erfindungsgemäßen Mischungen die Bedeutung 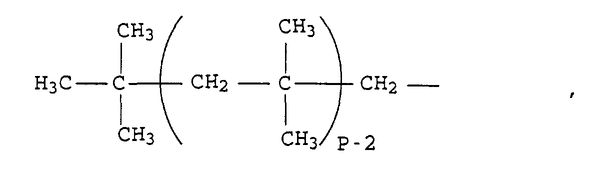 wobei der Polymerisationsgrad P = 10 bis 100 beträgt, und die Reste R2 und R6 für Methyl stehen.

Neben den Hauptprodukten XII bis XIV kann die erfindungsgemäße Mischung noch folgende Verbindungen als Nebenprodukte enthalten:

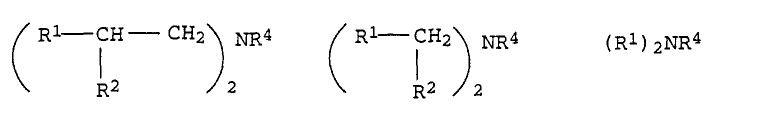

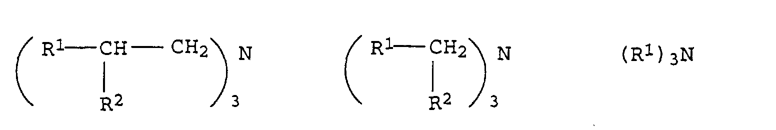

Als weitere Nebenprodukte finden sich manchmal Alkohole der Formel R¹-CHR²-CH₂OH, R¹-CHR²-OH und/oder R¹-OH.

Die nach dem erfindungsgemäßen Verfahren hergestellten Aminoalkane, Alkyloxime, Alkylnitrone und Mischungen hieraus, insbesondere jedoch die erfindungsgemäßen Mischungen mit den Strukturen XII bis XIV als Hauptkomponenten sowie die Verbindungen IX bis XVI selbst, eignen sich in hervorragender Weise als Additive für Kraft- und Schmierstoffe.

Werden die beschriebenen Produkte in Kraftstoffen eingesetzt, so gibt man sie bevorzugt in einer Menge von 10 bis 5000 ppm, insbesondere 50 bis 1000 ppm zu. In Schmierstoffen muß in der Regel höher additiviert werden, die Mengen können hier 0,1 bis 6 Gew.-%, insbesondere 0,5 bis 5 Gew.-%, betragen.

Sollen in erster Linie die dispergierenden Eigenschaften der Produkte genutzt werden, so kann man sie auch mit herkömmlichen Detergentien als zusätzlichen Additiven kombinieren.

Als Detergents-Komponente in der Mischung mit den erfindungsgemäßen Stoffen als Dispergatoren kann prinzipiell jedes bekannte der hierfür geeigneten Produkte eingesetzt werden, wie sie z.B. bei J. Falbe, U. Hasserodt, Katalysatoren, Tenside und Mineralöladditive, G. Thieme Verlag Stuttgart, 1978, S. 223 f., oder bei K. Owen, Gasoline and Diesel Fuel Additives, John Wiley & Sons, 1989, S. 23 ff., beschrieben sind.

Vorzugsweise verwendet man N-haltige Detergentien, z.B. Verbindungen, die eine Amin- oder Amid-Gruppe enthalten. Insbesondere geeignet sind Polyisobutylamine gemäß EP-A 244 616, Ethylendiamintetraessigsäureamide und/oder -imide gemäß EP-A 356 725, wobei auf die Definitionen in diesen Literaturstellen Bezug genommen wird. Die dort beschriebenen Produkte verfügen herstellungsbedingt ebenfalls - wie die beschriebenen Produkte - über den Vorteil, chlor- bzw. chloridfrei zu sein.

Soll in erster Linie die Detergents-Wirkung der erfindungsgemäßen Umsetzungsprodukte genutzt werden, so können diese Stoffe auch mit Trägerölen kombiniert werden. Derartige Trägeröle sind bekannt, insbesondere eignen sich Trägeröle auf Polyglykolbasis, z.B. entsprechende Ether und/oder Ester, wie sie in der US-A 5 004 478 oder der DE-A 38 38 918 beschrieben sind. Auch Polyoxyalkylenmonoole mit Kohlenwasserstoffendgruppen (US-A 4 877 416) oder Trägeröle, wie sie in der DE-A 41 42 241 offenbart sind, können eingesetzt werden.

Als Kraftstoffe für Ottomotoren kommen verbleites und insbesondere unverbleites Normal- und Superbenzin in Betracht. Die Benzine können auch andere Komponenten als Kohlenwasserstoffe, z.B. Alkohole wie Methanol, Ethanol oder tert.-Butanol sowie Ether, z.B. Methyl-tert.-butylether, enthalten. Neben den erfindungsgemäßen Umsetzungsprodukten enthalten die Kraftstoffe in der Regel noch weitere Zusätze wie Korrosionsinhibitoren, Stabilisatoren, Antioxidantien und/oder weitere Detergentien.

Korrosionsinhibitoren sind meist Ammoniumsalze organischer Carbonsäuren, die durch entsprechende Struktur der Ausgangsverbindungen zur Filmbildung neigen. Auch Amine zur Absenkung des pH-Wertes finden sich häufig in Korrosionsinhibitoren. Als Buntmetallkorrosionsschutz werden meist heterocyclische Aromaten eingesetzt.

Die Prüfung der Produkte auf Eignung als Kraftstoffadditive erfolgte mittels Motorentests; in Prüfstandsversuchen gemäß CEC-F-05-T-92 wurde die keep-clean-Wirkung bei Einlaßventilen (Mercedes-Benz M 102 E-Motor) getestet.

### Herstellungsbeispiele

### Beispiel 1

In einem 300-ml-Druckautoklav wurden 10 g Reaktionsprodukt aus hochreaktivem Polyisobuten (Glissopal® ES 3250) und Stickstoffdioxid (gemäß Beispiel 4 von (1)), 80 ml Tetrahydrofuran, 15 ml Methanol und 5 g Raney-Nickel eingefüllt. Durch 2maliges Aufpressen von 20 bar Stickstoff und nachfolgendem Entspannen wurde der Gasraum von Sauerstoff befreit. Nach der Zugabe von 15 g Ammoniak wurden 100 bar Wasserstoff aufgepreßt und es wurde auf 200°C erhitzt. Wasserstoff wurde auf 200 bar nachgepreßt. Der Autoklaveninhalt wurde 10 Stunden bei 200°C und 200 bar gerührt. Der abgekühlte Austrag wurde filtriert und das Filtrat im Vakuum eingedampft. Man erhielt 7 g Rückstand mit einer Aminzahl von 53 mg KOH/g.

Das Produkt bestand in der Hauptsache aus X¹NH₂ und X²NH₂ neben X³NH₂ und sekundären Aminanteilen in Spuren (X¹NH₂ : 30 Gew.-%, X²NH₂ : 60 Gew.-%, X³NH₂ : 10 Gew.-%).

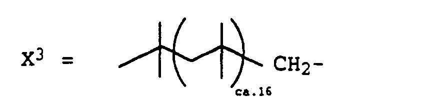

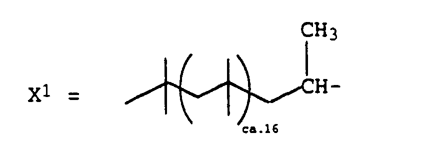

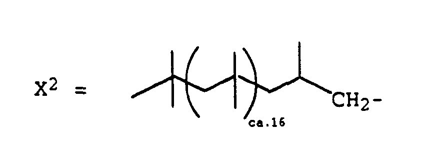

### Beispiel 2

In einem 3,5-l-Druckautoklav wurden 180 g Reaktionsprodukt aus hochreaktivem Polyisobuten (Glissopal ES 3250) und Stickstoffdioxid (gemäß Beispiel 4 von (1)), 900 g Mihagol® M (n-Alkangemisch C₁₀-C₁₄) und 45 g Raney-Nickel eingefüllt. Durch 2maliges Aufpressen von 20 bar Stickstoff und nachfolgendem Entspannen wurde der Gasraum von Sauerstoff befreit. Anschließend wurden 70 bar Wasserstoff aufgepreßt und es wurde auf 200°C erhitzt. Wasserstoff wurde auf 200 bar nachgepreßt und der Autoklav 10 Stunden bei 200°C und 200 bar gerührt. Nach dem Abkühlen wurde das Reaktionsgemisch filtriert und im Vakuum wurden Lösungsmittel und der bei der Reaktion gebildete Ammoniak abdestilliert. Es verblieben 168 g eines Öls mit der Aminzahl 36 mg KOH/g und einer OH-Zahl von 8 mg KOH/g.

Das Produkt bestand in der Hauptsache aus X¹NH₂ und X²NH₂ neben X³NH₂ und X¹OH.

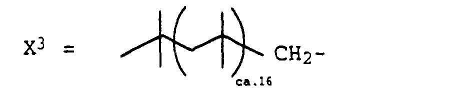

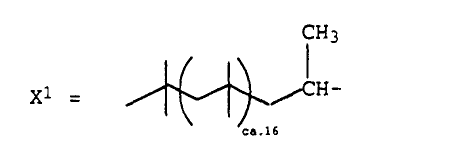

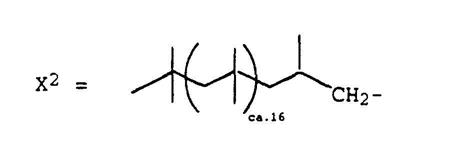

### Beispiel 3

a) In einem 200-ml-Rührbehälter wurden 20 g Reaktionsprodukt aus hochreaktivem Polyisobuten(Glissopal ES 3250) und Stickstoffdioxid (gemäß Beispiel 4 von (1)), 100 ml Tetrahydrofuran und 6,5 g 3-Dimethylaminopropylamin eingefüllt und 3 Stunden bei 40°C gerührt. Anschließend wurden die leichtflüchtigen Bestandteile im Vakuum abdestilliert. Es verblieben 21 g öliger Rückstand, der neben Resten von 3-Dimethylaminopropylamin als Hauptprodukt das 3-Dimethylaminopropylimin eines Keto-polyisobutens enthielt: 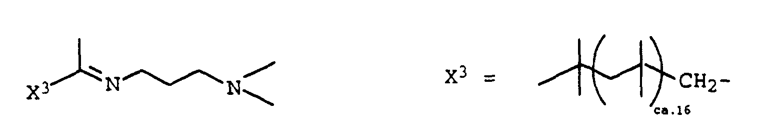
b) 20 g des öligen Rückstandes wurden mit 100 ml Tetrahydrofuran und 5 g Raney-Nickel in einen 300 ml Druckautoklaven gefüllt. Durch 2maliges Aufpressen von 20 bar Stickstoff und nachfolgendem Entspannen wurde der Gasraum von Sauerstoff befreit. Nach dem Aufpressen von 50 bar Wasserstoff wurde auf 150°C erhitzt, der Druck mit Wasserstoff auf 100 bar erhöht und bei diesen Bedingungen 10 Stunden gerührt. Nach dem Abkühlen wurde vom Nickel abfiltriert und die flüchtigen Anteile wurden im Vakuum, zuletzt bei 1 mbar und 120°C, abdestilliert. Man erhielt 16,9 g Rückstand mit einer Aminzahl von 77 mg KOH/g.

Das Produkt bestand zu ca. 75 Gew.-% aus

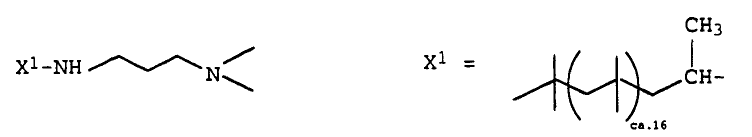

### Beispiel 4

In eine 1-l-Rührapparatur, die mit Stickstoff gespült war, wurden 100 ml trockenes Tetrahydrofuran und 4,6 g Lithiumaluminiumhydrid eingefüllt. Anschließend tropfte man eine Lösung, bestehend aus 30 g Reaktionsprodukt aus hochreaktivem Polyisobuten (Glissopal ES 3250) und Stickstoffdioxid (gemäß Beispiel 4 von (1)) und trockenem 100 ml Tetrahydrofuran in dem Maße zu, daß sich die Temperatur im Kolben bei 40 bis 50°C hielt. Nach dem Zutropfen wurde 2 Stunden bei Zimmertemperatur gerührt und anschließend das überschüssige Lithiumaluminiumhydrid vorsichtig mit Wasser hydrolysiert. Das Reaktionsgemisch wurde über Kieselgur abgesaugt und das Filtrat im Vakuum eingeengt. Man erhielt 26,5 g Rückstand mit einer Aminzahl von 33 mg KOH/g.

Das Produkt besteht zu ca. 75 Gew.-% aus X²NH₂ und zu ca. 25 Gew.-% aus X¹OH:

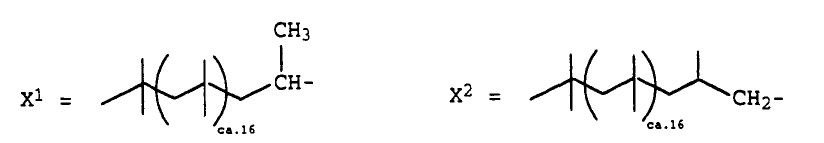

### Beispiel 5

In einen 0,5-l-Druckautoklav wurden 50 g Reaktionsprodukt aus hochreaktivem Polyisobuten (Glissopal ES 3250) und Stickstoffdioxid (gemäß Beispiel 4 von (1)), 200 ml Tetrahydrofuran und 10 g Katalysator HO-50 (50 Gew.-% Wasser; 2,5 Gew.-% Palladium; 47,5 Gew.-% Kohle) eingefüllt. Durch 2maliges Aufpressen von 5 bar Wasserstoff und nachfolgendem Entspannen wurde der Gasraum von Sauerstoff befreit. Es wurde auf 50°C erwärmt und mit Wasserstoff auf 7 bar nachgepreßt. Bei 50°C und 7 bar wurde der Autoklaveninhalt 7 Stunden gerührt. Dabei fand eine Wasserstoff-Aufnahme von 22,3 bar (entsprechend 5,6 l) statt. Das abgekühlte Reaktionsgemisch wurde filtriert und das Filtrat eingedampft.

### Der Rückstand bestand in der Hauptsache aus den Substanzen

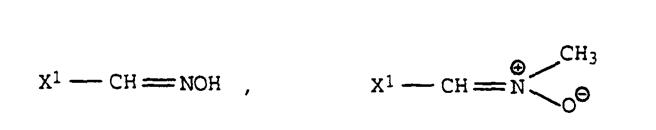

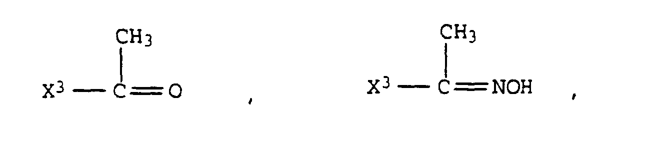

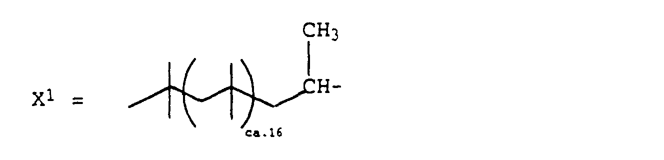

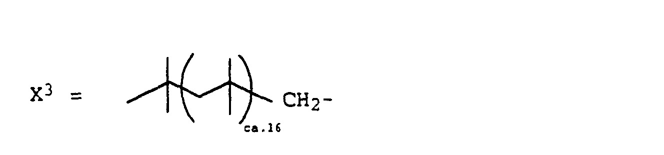

### Beispiel 6

In einen 300-ml-Druckautoklaven wurden 20 g Reaktionsprodukt aus hochreaktivem Polyisobuten (Glissopal ES 3250) und Stickstoffdioxid (gemäß Beispiel 4 von (1)), 100 ml Tetrahydrofuran und 5 g Raney-Nickel eingefüllt. Durch 2maliges Aufpressen von 20 bar Stickstoff und nachfolgendem Entspannen wurde der Gasraum von Sauerstoff befreit. Nach der Zugabe von 22 g Methylamin wurden 50 bar Wasserstoff aufgepreßt und auf 200°C erhitzt. Wasserstoff wurde auf 200 bar nachgepreßt und der Autoklav 10 Stunden bei 200°C und 200 bar gerührt. Das abgekühlte Reaktionsgemisch wurde filtriert und das Filtrat im Vakuum eingedampft. Man erhielt 17,4 g Rückstand mit einer Aminzahl von 46 mg KOH/g.

Das Produkt bestand in der Hauptsache aus: X³NHCH₃, X¹NHCH₃, X₂NHCH₃ sowie X¹NH₂

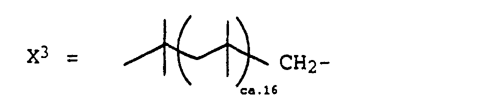

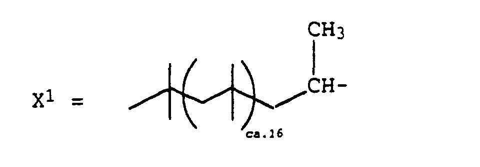

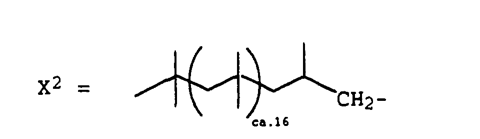

### Beispiel 7

In einen 300-ml-Druckautoklaven wurden 20 g Reaktionsprodukt aus hochreaktivem Polyisobuten (Glissopal ES 3250) und Stickstoffdioxid (gemäß Beispiel 4 von (1)), 100 ml Tetrahydrofuran und 5 g Raney-Nickel eingefüllt. Durch 2maliges Aufpressen von 20 bar Stickstoff und nachfolgendem Entspannen wurde der Gasraum von Sauerstoff befreit. Nach Zugabe von 32 g Dimethylamin wurden 50 bar Wasserstoff aufgepreßt und es wurde auf 200°C erhitzt. Wasserstoff wurde auf 200 bar nachgepreßt und der Autoklav 10 Stunden bei 200°C und 200 bar gerührt. Das abgekühlte Reaktionsgemisch wurde filtriert und das Filtrat im Vakuum eingedampft. Man erhielt 16 g Rückstand mit einer Aminzahl von 40 mg KOH/g.

Das Produkt besteht in der Hauptsache aus:

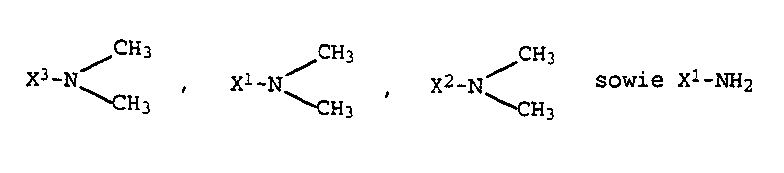

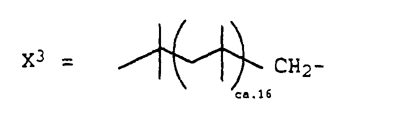

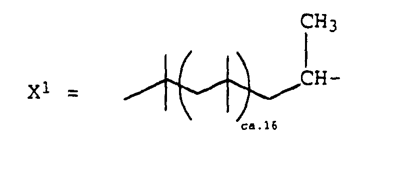

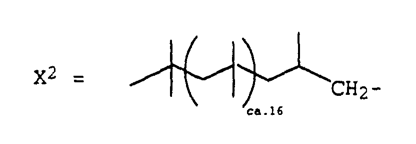

### Beispiel 8

In eine 0,5-l-Rührapparatur, die mit Stickstoff gespült worden war, wurden 100 ml trockenes Tetrahydrofuran und 4 g Lithiumaluminiumhydrid eingefüllt. Anschließend tropfte man eine Lösung, bestehend aus 100 ml Tetrahydrofuran und 50 g Nitro-polyisobuten (gemäß Beispiel 8 von (1)), welches erhalten wird, wenn das Reaktionsprodukt aus hochreaktivem Polyisobuten (Glissopal ES 3250) und Stickstoffdioxid (gemäß Beispiel 4 von (1)) mit einer wäßrigen Natriumcarbonat-Lösung gerührt wird und neben (XV) aus (XVI) besteht, in dem Maße zu, daß sich die Temperatur im Kolben bei 45 bis 50°C hielt.Nach dem Zutropfen wurde 3 Stunden bei Zimmertemperatur gerührt und anschließend vorsichtig mit 5 g Wasser hydrolysiert. Das Reaktionsgemisch wurde über Kieselgur abgesaugt und das Filtrat im Vakuum eingeengt.

Man erhielt 41 g Ö1 mit der Aminzahl 33 mg KOH/g und der gleichen Zusammensetzung wie das Produkt aus Beispiel 4, also ca.
75 Gew.-% X²NH₂ und ca. 25 Gew.-% X¹OH.

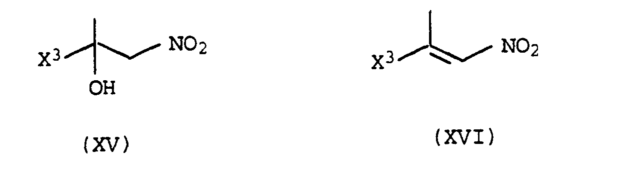

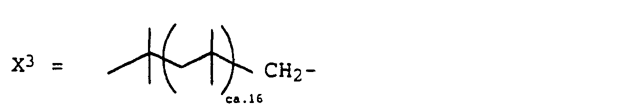

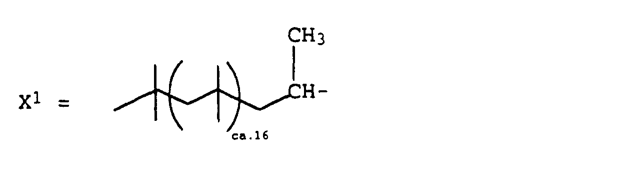

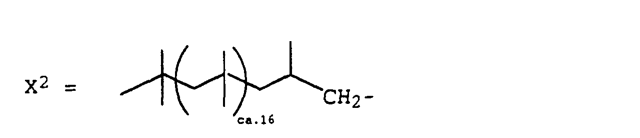

### Beispiel 9

In einen 300-ml-Druckautoklaven wurden 10 g Keto-polyisobuten der Formel X³-CO-CH₃, welches aus dem Reaktionsprodukt aus hochreaktivem Polyisobuten (Glissopal ES 3250) und Stickstoffdioxid (gemäß Beispiel 4 von (1)) entsteht, indem man letzteres mit einer wäßrigen Ammoniaklösung bei 25°C rührt, 80 ml Tetrahydrofuran 15 ml Methanol und 5 g Raney-Nickel eingefüllt. Durch 2maliges Aufpressen von 20 bar Stickstoff und nachfolgendem Entspannen wurde der Gasraum von Sauerstoff befreit. Nach der Zugabe von 12,5 g Ammoniak wurden 70 bar Wasserstoff aufgepreßt und es wurde auf 200°C erhitzt. Wasserstoff wurde auf 200 bar nachgepreßt und der Autoklav 10 Stunden bei 200°C und 200 bar gerührt. Das abgekühlte Reaktionsgemisch wurde filtriert und das Filtrat im Vakuum eingedampft. Man erhielt 8,8 g Rückstand mit einer Aminzahl von 43,5 mg KOH/g.

Das Produkt bestand größtenteils aus X¹NH₂.

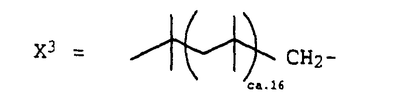

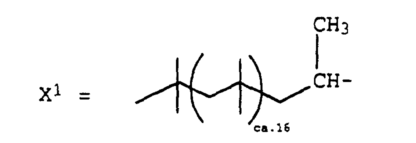

### Beispiel 10

In einen 300-ml-Druckautoklaven wurden 20 g Keto-polyisobuten der Formel X³-CO-CH₃, welches aus dem Reaktionsprodukt aus hochreaktivem Polyisobuten (Glissopal ES 3250) und Stickstoffdioxid (gemäß Beispiel 4 von (1)) entsteht, indem man letzteres mit einer wäßrigen Ammoniaklösung bei 25°C rührt, 100 ml Tetrahydrofuran, 6,2 g 3-Dimethylaminopropylamin und 5 g Raney-Nickel eingefüllt. Durch 2maliges Aufpressen von 20 bar Stickstoff und nachfolgendem Entspannen wurde der Gasraum von Sauerstoff befreit. Anschließend wurden 50 bar Wasserstoff aufgepreßt und es wurde auf 150°C erhitzt. Wasserstoff wurde auf 100 bar nachgepreßt und 10 Stunden bei 150°C und 100 bar gerührt. Der abgekühlte Autoklaveninhalt wurde filtriert und das Filtrat im Vakuum, zuletzt bei 1 mbar und 80°C eingedampft. Man erhielt 20 g Rückstand mit einer Aminzahl von 108 mg KOH/g, die sich aufteilt in 12 mg KOH/g für primäre, 44 mg KOH/g für sekundäre und 51 mg KOH/g für tertiäre Aminanteile.

Das Produkt bestand aus:

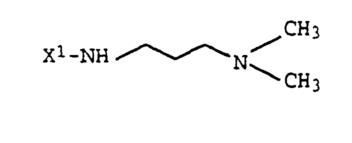

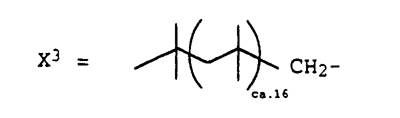

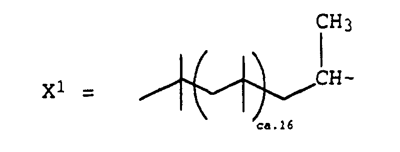

### Beispiele 11a bis e

In einem 300 ml-Druckautoklav wurden 20 g Reaktionsprodukt aus hochreaktivem Polyisobuten (Glissopal ES 3250) und Stickstoffdioxid (gemäß Beispiel 4 von (1)), 20 g Mihagol M (n-Alkangemisch C₁₀-C₁₄) 100 g Tetrahydrofuran und 2,5 g bzw. 5 g Katalysator gemäß nachfolgender Tabelle eingefüllt. Durch 2maliges Aufpressen von 20 bar Stickstoff und nachfolgendem Entspannen wurde der Gasraum von Sauerstoff befreit. Nach der Zugabe von 15 g Ammoniak wurden 100 bar Wasserstoff aufgepreßt und es wurde auf 200°C erhitzt. Wasserstoff wurde auf 200 bar nachgepreßt. Der Autoklaveninhalt wurde 10 Stunden bei 200°C und 200 bar gerührt. Der abgekühlte Austrag wurde filtriert und das Filtrat im Vakuum eingedampft. Man erhielt 17 g Rückstand mit einer Aminzahl von 30 bis 50 mg KOH/g (s. Tabelle).

Das Produkt bestand bei allen 5 Beispielen in der Hauptsache aus X¹NH₂ und X²NH₂ neben X³NH₂ und gegebenenfalls sekundären Aminanteilen (maximal 5 Gew.-%).

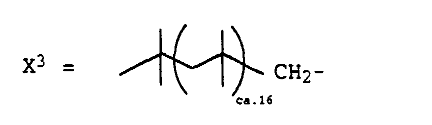

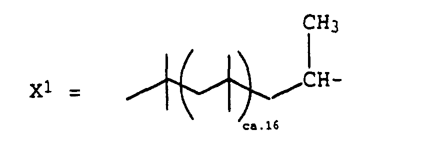

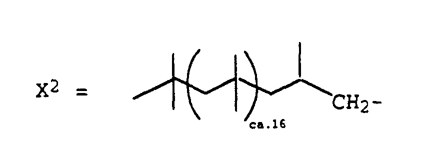

**Tabelle**

| zu Beispiel 11a bis e | | | | | |
|---|---|---|---|---|---|
| Beispiel | Katalysator | Aminzahl | Produktzusammensetzung [Gew.-%] | | |
| | | | X¹NH₂ | X²NH₂ | X³NH₂ |
| 11a | 5 g Ruthenium auf Kohle 5 gew.-%ig; wasserfeucht (50 Gew.-%) | 52 | 75 | 10 | 10 |
| 11b | 2,5 g Rhodium auf Kohle 5 gew.%ig; trocken | 52 | 30 | 55 | 10 |
| 11c | 5 g Platin auf Kohle 5%ig; wasserfeucht (50 Gew.-%) | 33 | 30 | 60 | 5 |
| 11d | 5 g H 0-50: Palladium auf Kohle 5 gew.-%ig; wasserfeucht (50 Gew.-%) | 50 | 50 | 40 | 10 |
| 11e | 5 g H 1-88: handelsüblicher Mischkatalysator (Ni, Zr, Cu, Mo) | 48 | 40 | 55 | 5 |

### Beispiel 12

Das Reaktionsgemisch aus Beispiel 5 wurde mit 100 ml Tetrahydrofuran und 10 g Raney-Nickel in einen 300 ml Druckautoklav gefüllt. Durch 2maliges Aufpressen von 20 bar Stickstoff und nachfolgendem Entspannen wurde der Gasraum von Sauerstoff befreit. Nach der Zugabe von 15 g Ammoniak wird auf 100°C erwärmt und Wasserstoff auf 100 bar aufgepreßt. Der Autoklav wird 10 Stunden bei 100°C und 100 bar gerührt. Nach dem Abkühlen wurde das Reaktionsgemisch filtriert und das Filtrat eingedampft. Es verblieben 24 g Rückstand mit einer Aminzahl von 32 mg KOH/g.

### Beispiel 13

In einen 300-ml-Druckautoklaven wurden 10 g Reaktionsprodukt aus Polyisobuten mit einem hohen Anteil an β-ständigen Doppelbindungen (Indopol® H100) und Stickstoffdioxid (gemäß 2 von (2)), 80 ml Tetrahydrofuran und 15 ml Methanol und 5 g Raney-Nickel eingefüllt. Durch zweimaliges Aufpressen von 20 bar Stickstoff und nachfolgendem Entspannen wurde der Gasraum von Sauerstoff befreit. Nach der Zugabe von 15 g Ammoniak wurden 70 bar Wasserstoff aufgepreßt und es wurde auf 200°C erhitzt. Wasserstoff wurde auf 200 bar nachgepreßt und der Autoklav 10 Stunden bei 200°C und 200 bar gerührt. Das abgekühlte Reaktionsgemisch wurde abfiltriert und das Filtrat im Vakuum eingedampft. Man erhielt 8,2 g Rückstand mit einer Aminzahl von 56 mg KOH/g.

### Beispiel 14

In einen 3,5-l-Druckautoklaven wurden 150 g Reaktionsprodukt aus Oligopropen (mittleres Molgewicht ca. 378, Br-Zahl 43, vinyliden-terminiert) und Stickstoffdioxid, 1000 ml Tetrahydrofuran und 38 g Raney-Nickel eingefüllt. Durch 2maliges Aufpressen von 20 bar Stickstoff und nachfolgendem Entspannen wurde der Gasraum von Sauerstoff befreit. Nach der Zugabe von 90 g Ammoniak wurden 100 bar Wasserstoff aufgepreßt und es wurde auf 200°C erhitzt. Wasserstoff wurde auf 200 bar nachgepreßt und der Autoklav 10 Stunden bei 200°C und 200 bar gerührt. Das abgekühlte Reaktionsgemisch wurde filtriert und das Filtrat eingedampft. Man erhielt 118 g Rückstand mit einer Aminzahl von 127,4 mg KOH/g, Stickstoffgehalt 3,8 Gew.-%.

Das Produkt bestand hauptsächlich aus Y¹NH₂ und Y²NH₂.

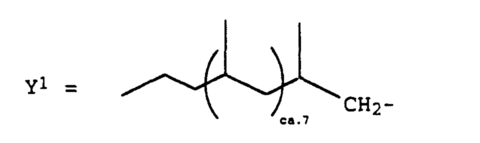

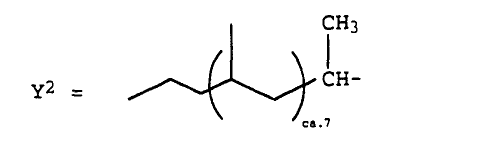

### Beispiel 15

In eine 0,5-l-, mit Stickstoff gespülte Rührapparatur wurden 100 ml trockenes Tetrahydrofuran und 8 g Lithiumaluminiumhydrid eingefüllt. Anschließend tropfte man eine Lösung, bestehend aus 25 g Reaktionsprodukt aus Oligopropen (mittleres Molgewicht ca. 378, Bromzahl 43, vinyliden-terminiert) und Stickstoffdioxid und 100 ml Tetrahydrofuran in dem Maße zu, daß sich die Temperatur im Kolben bei ca. 40°C hielt. Nach dem Zutropfen wurde 3 Stunden bei Zimmertemperatur gerührt und anschließend das überschüssige Lithiumaluminiumhydrid mit wenig Wasser vorsichtig hydrolysiert. Das Reaktionsgemisch wurde über Kieselgur abgesaugt und das Filtrat im Vakuum eingedampft. Man erhielt 18,2 g Rückstand mit einer Aminzahl von 84,4.

Das Produkt bestand hauptsächlich aus Y¹NH₂ und Y²-OH.

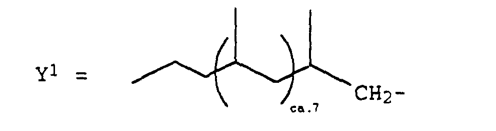

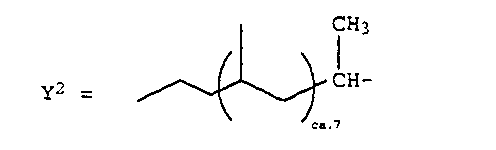

### Beispiel 16

In einen 300-ml-Druckautoklaven wurden 20 g Reaktionsprodukt aus Oligopropen (mittleres Molgewicht ca. 336, Bromzahl 47,5, zu ca. 90 % vinyl-terminiert) und Stickstoffdioxid (in Analogie zu Beispiel 1 von (2)), 100 ml Tetrahydrofuran und 5 g Raney-Nickel eingefüllt. Durch zweimaliges Aufpressen von 20 bar Stickstoff und nachfolgendem Entspannen wurde der Gasraum von Sauerstoff befreit. Nach der Zugabe von 20 g Ammoniak werden 50 bar Wasserstoff aufgepreßt und es wurde auf 200°C erhitzt. Wasserstoff wurde auf 200 bar nachgepreßt und der Autoklav 10 Stunden bei 200°C und 200 bar gerührt. Das abgekühlte Reaktionsgemisch wurde abfiltriert und im Vakuum abgedampft. Man erhielt 15,5 g Rückstand mit einer Aminzahl von 153 mg KOH/g und einem Stickstoffwert von 4,4 Gew.-%.

Das Produkt bestand in der Hauptsache aus Z¹NH₂.

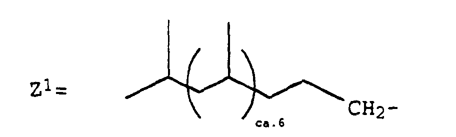

### Beispiel 17

In eine 0,5-l-, mit Stickstoff gespülte Rührapparatur wurden 100 ml trockenes Tetrahydrofuran und 8 g Lithiumaluminiumhydrid eingefüllt. Anschließend tropfte man eine Lösung, bestehend aus 25 g Reaktionsprodukt aus Oligopropen (mittleres Molgewicht ca. 336, Bromzahl 47,5, zu ca. 90 % vinyl-terminiert) und Stickstoffdioxid (in Analogie zu Beispiel 1 aus (2)) und 100 ml Tetrahydrofuran in dem Maße zu, daß sich die Temperatur im Kolben bei 40°C hielt. Nach dem Zutropfen wurden 3 Stunden bei Zimmertemperatur gerührt und anschließend der Reaktionsansatz mit wenig Wasser vorsichtig hydrolysiert. Das Reaktionsgemisch wurde über Kieselgur abgesaugt und das Filtrat im Vakuum eingedampft. Man erhielt 18 g Rückstand mit einer Aminzahl von 84,3.

Das Produkt bestand hauptsächlich aus Z¹NH₂ und Z²NH₂.

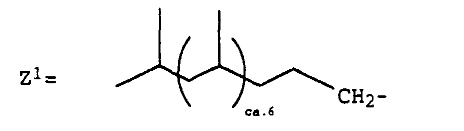

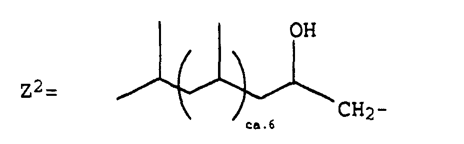

Beispiel 17 dient lediglich zu Vergleichszwecken und veranschaulicht eine Ausführungsform von (2).

### Anwendungsbeispiele

### Keep-clean-test bei Einlaßventilen

Die Motorversuche wurden mit einem Mercedes-Benz M 102 E-Motor (nach CEC-F-05-T-92) durchgeführt:

### Eingesetzter Kraftstoff: Euro-Super bleifrei

| Additiv | | Einlaßventilablagerungen [mg] | | | | |
|---|---|---|---|---|---|---|
| | Dosierung [ppm] | Ventil 1 | Ventil 2 | Ventil 3 | Ventil 4 | Durchschnitt |
| Produkt aus Beispiel 1 | 300 | 45 | 48 | 50 | 131 | 69 |
| zum Vergleich: Dinitroalkan gemäß Bsp.4 von (1) | 400 | 187 | 31 | 166 | 145 | 132 |
| ohne (Blindwert) | - | 309 | 420 | 312 | 303 | 336 |

Die Ergebnisse zeigen deutlich die hervorragende ventilreinigende Wirkung der erfindungsgemaßen Additive, die schon bei relativ niedriger Dosierrate zu Tage tritt.

## Patentansprüche

1. Verfahren zur Herstellung von organischen Stickstoffverbindungen, welche nur eine stickstoffunktionelle Gruppierung und keine alkoholischen Hydroxylgruppen im Molekül tragen, aus Nitrogruppen enthaltenden Umsetzungsprodukten von Polymerisaten von C₂- bis C₆-Olefinen mit einem mittleren Polymerisationsgrad P = 5 bis 100 mit Stickoxiden oder Gemischen aus Stickoxiden und Sauerstoff, dadurch gekennzeichnet, daß man die Nitrogruppen enthaltenden Umsetzungsprodukte direkt im Anschluß an ihre Bildung aus den Olefinpolymerisaten und den Stickoxiden hydriert.

2. Verfahren nach Anspruch 1 zur Herstellung von Aminoalkanen, Alkyloximen, Alkylnitronen oder Mischungen hieraus als organische Stickstoffverbindungen.

3. Verfahren zur Herstellung von organischen Stickstoffverbindungen, welche nur eine stickstoffunktionelle Gruppierung und keine alkoholischen Hydroxylgruppen im Molekül tragen, aus Nitrogruppen enthaltenden Umsetzungsprodukten von Polymerisaten von C₂- bis C₆-Olefinen mit einem mittleren Polymerisationsgrad P = 5 bis 100 mit Stickoxiden oder Gemischen aus Stickoxiden und Sauerstoff, dadurch gekennzeichnet, daß man die Nitrogruppen enthaltenden Umsetzungsprodukte im Anschluß an ihre Bildung durch Eliminierung mit Basen in Nitrogruppen enthaltende Alkene umwandelt und diese danach hydriert, wobei resultierende Aminoalkane immer in Form von Mischungen von Verbindungen mit unterschiedlicher Anzahl von C-Atomen anfallen.

4. Verfahren zur Herstellung von organischen Stickstoffverbindungen, welche nur eine stickstoffunktionelle Gruppierung und keine alkoholischen Hydroxylgruppen im Molekül tragen, aus Nitrogruppen enthaltenden Umsetzungsprodukten von Polymerisaten von C₂- bis C₆-Olefinen mit einem mittleren Polymerisationsgrad P = 5 bis 100 mit Stickoxiden oder Gemischen aus Stickoxiden und Sauerstoff, dadurch gekennzeichnet, daß man die Nitrogruppen enthaltenden Umsetzungsprodukte in Verbindungen mit reaktiven Carbonylfunktionen überführt, diese mit Ammoniak oder primären Aminen zu Iminen umsetzt, wobei die resultierenden organischen Stickstoffverbindungen, herrührend von den eingesetzten primären Aminen, auch mehrere stickstoffunktionelle Gruppen und alkoholische Hydroxylgruppen im Molekül tragen können, und anschließend hydriert.

5. Verfahren nach den Ansprüchen 1 bis 4, dadurch gekennzeichnet, daß man die Hydrierung als katalytische Hydrierung mit Wasserstoff in Gegenwart von Hydrierungskatalysatoren, als Transferhydrierung mit reduzierend wirkenden organischen oder anorganischen Molekülverbindungen, als Reduktion mit unedlen Metallen oder als Reduktion mit salzartigen komplexen Hydriden oder salzartigen niedervalenten Schwefelverbindungen durchführt.

6. Verfahren nach den Ansprüchen 1 bis 5, dadurch gekennzeichnet, daß man die Hydrierung in Gegenwart von Ammoniak oder primären, sekundären oder tertiären Aminen, Diaminen, Polyaminen, Alkanolaminen, Etheraminen, Polyetheraminen oder Polyetheralkanolaminen durchführt, wobei die resultierenden organischen Stickstoffverbindungen, herrührend von den eingesetzten Aminen und Aminderivaten, auch mehrere stickstoffunktionelle Gruppen und alkoholische Hydroxylgruppen im Molekül tragen können.

7. Verfahren nach den Ansprüchen 1 bis 6, dadurch gekennzeichnet, daß man die Hydrierung unter neutralen oder basischen Reaktionsbedingungen durchführt.

8. Verfahren nach den Ansprüchen 1 bis 7, dadurch gekennzeichnet, daß man die Hydrierung bei Temperaturen von 20 bis 250°C durchführt.

9. Verfahren nach den Ansprüchen 1 bis 8, wobei als Nitrogruppen enthaltende Umsetzungsprodukte solche von Polymerisaten von Isobuten mit einem mittleren Polymerisationsgrad P = 5 bis 100, bei denen bis zu 50 Gew.-% des Isobutens durch andere C₂- bis C₆-Olefine als Comonomere ersetzt sein können, mit Stickoxiden oder Gemischen aus Stickoxiden und Sauerstoff eingesetzt werden.

10. Verfahren nach den Ansprüchen 1 bis 8, wobei als Nitrogruppen enthaltende Umsetzungsprodukte solche von Polymerisaten von C₂- bis C₆-Olefinen mit einem mittleren Polymerisationsgrad P = 5 bis 100 und Stickoxiden oder Gemischen aus Stickoxiden und Sauerstoff, die in Form einer Mischung verschiedener Nitrogruppen enthaltender Alkane vorliegen und als Hauptkomponenten die Verbindung der allgemeinen Formel I und II 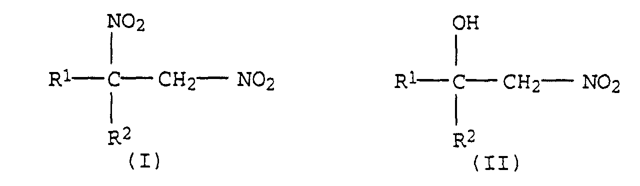 in denen
R¹ einen langkettigen linearen oder verzweigten Alkylrest mit 8 bis 600 C-Atomen bezeichnet und
R² für Wasserstoff oder C₁- bis C₃-Alkyl steht,
enthalten, eingesetzt werden.

11. Verfahren nach den Ansprüchen 1 bis 8, wobei als Nitrogruppen enthaltende Umsetzungsprodukte solche von Polymerisaten von C₂- bis C₆-Olefinen mit einem mittleren Polymerisationsgrad P = 5 bis 100 und einem hohen Anteil an β-ständigen und einem geringen Anteil an endständigen Doppelbindungen mit Stickoxiden oder Gemischen aus Stickoxiden und Sauerstoff, die in Form einer Mischung verschiedener Nitrogruppen enthaltender Alkane vorliegen und als Hauptkomponenten die Verbindungen der allgemeinen Formel III und IV 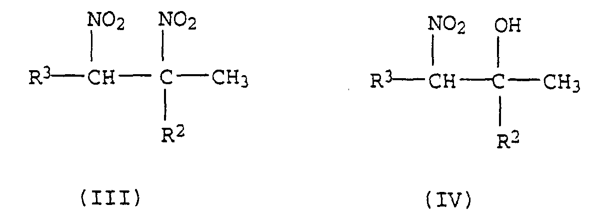 in denen
R³ einen langkettigen linearen oder verzweigten Alkylrest mit 8 bis 600 C-Atomen bezeichnet und
R² für Wasserstoff oder C₁- bis C₃-Alkyl steht,
enthalten, eingesetzt werden.

12. Verfahren nach den Ansprüchen 1 bis 8, wobei als Nitrogruppen enthaltende Umsetzungsprodukte solche von Polyisobutenen mit einem mittleren Polymerisationsgrad P = 10 bis 100 mit einem Anteil E = 60 bis 90 % an Doppelbindungen, die mit Maleinsäureanhydrid umsetzbar sind, wobei E = 100 % dem rechnerisch-theoretischen Wert für den Fall entspräche, daß jedes Molekül des Polyisobutens eine derartige reaktive Doppelbindung hätte, mit Stickoxiden oder Gemischen aus Stickoxiden und Sauerstoff eingesetzt werden.

13. Verfahren nach den Ansprüchen 1 bis 8, wobei als Nitrogruppen enthaltende Umsetzungsprodukte solche von Polyisobutenen mit einem mittleren Polymerisationsgrad P = 10 bis 100 mit einem Anteil E = 60 bis 90 % an Doppelbindungen, die mit Maleinsäureanhydrid umsetzbar sind, wobei E = 100 % dem rechnerisch-theoretischen Wert für den Fall entspräche, daß jedes Molekül des Polyisobutens eine derartige reaktive Doppelbindung hätte, mit Stickoxiden oder Gemischen aus Stickoxiden und Sauerstoff, die in Form einer Mischung verschiedener Nitrogruppen enthaltender Alkane vorliegen und als Hauptkomponenten die Verbindungen der allgemeinen Formel V bis VIII 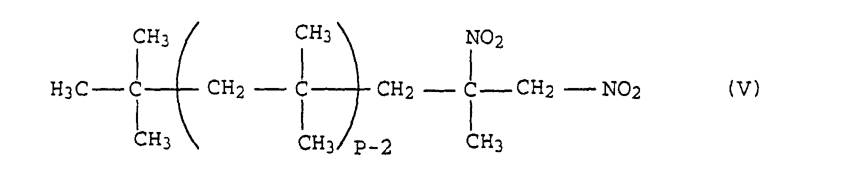 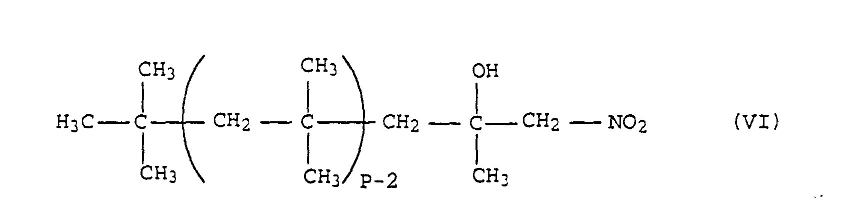 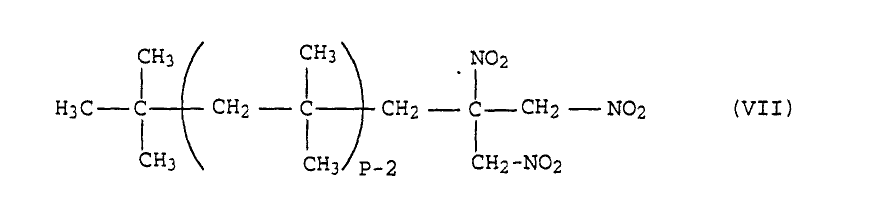 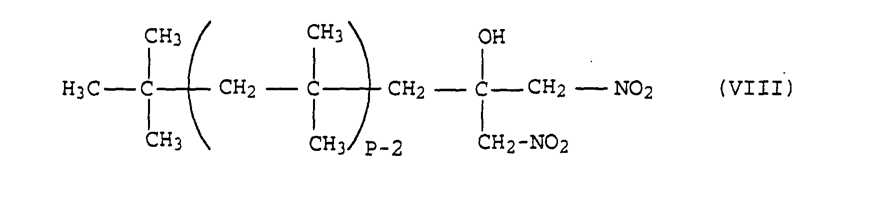 enthalten, eingesetzt werden.

14. Mischungen aus Alkyloximen und Alkylnitronen und gegebenenfalls Aminoalkanen mit den gleichen langkettigen Resten, welche als Hauptkomponenten die Verbindungen der allgemeinen Formel XII bis XIV 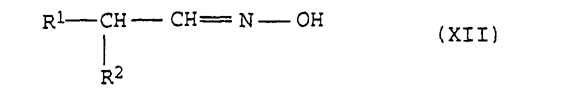 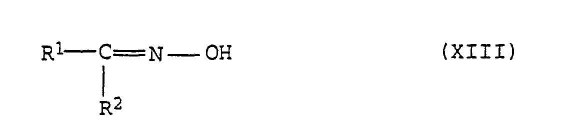 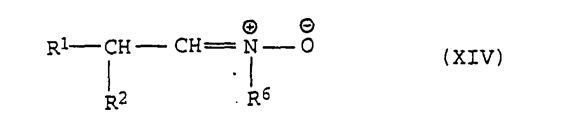 in denen
R¹ einen langkettigen linearen oder verzweigten Alkylrest mit 8 bis 600 C-Atomen bezeichnet,
R² für Wasserstoff oder C₁- bis C₃-Alkyl steht und
R⁶ C₁- bis C₃-Alkyl bedeutet,
enthalten.

15. Mischungen nach Anspruch 14, bei denen die drei angegebenen Hauptkomponenten im Gew.-Verhältnis von (1-98): (1-98) : (1-98) stehen.

## Claims

1. A process for preparing organic nitrogen compounds which have only one nitrogen-functional group and no alcoholic hydroxyl groups in the molecule from nitro-containing reaction products of polymers of C₂-C₆-olefins with an average degree of polymerization P = 5-100 with nitrogen oxides or mixtures of nitrogen oxides and oxygen, wherein the nitro-containing reaction products are hydrogenated directly after their formation from the olefin polymers and the nitrogen oxides.

2. A process as claimed in claim 1 for preparing aminoalkanes, alkyloximes, alkylnitrones or mixtures thereof as organic nitrogen compounds.

3. A process for preparing organic nitrogen compounds which have only one nitrogen-functional group and no alcoholic hydroxyl groups in the molecule from nitro-containing reaction products of polymers of C₂-C₆-olefins with an average degree of polymerization P = 5-100 with nitrogen oxides or mixtures of nitrogen oxides and oxygen, wherein the nitro-containing reaction products are, after their formation, converted by elimination with bases into nitro-containing alkenes and these are then hydrogenated, with the resulting aminoalkanes always being in the form of mixtures of compounds which differ in the number of carbon atoms.

4. A process for preparing organic nitrogen compounds which have only one nitrogen-functional group and no alcoholic hydroxyl groups in the molecule from nitro-containing reaction products of polymers of C₂-C₆-olefins with an average degree of polymerization P = 5-100 with nitrogen oxides or mixtures of nitrogen oxides and oxygen, wherein the nitro-containing reaction products are converted into compounds with reactive carbonyl functionalities and these are reacted with ammonia or primary amines to give imines, it also being possible for the resulting organic nitrogen compounds derived from the primary amines employed to have several nitrogen-functional groups and alcoholic hydroxyl groups in the molecule, and subsequently hydrogenated.

5. A process as claimed in any of claims 1 to 4, wherein the hydrogenation is carried out as catalytic hydrogenation with hydrogen in the presence of hydrogenation catalysts, as transfer hydrogenation with organic or inorganic compounds with a reducing action, as reduction with base metals or as reduction with salt-like complex hydrides or salt-like low-valency sulfur compounds.

6. A process as claimed in any of claims 1 to 5, wherein the hydrogenation is carried out in the presence of ammonia or primary, secondary or tertiary amines, diamines, polyamines, alkanolamines, ether amines, polyether amines or polyether alkanolamines, it also being possible for the resulting organic nitrogen compounds derived from the amines and amine derivatives employed to have several nitrogen-functional groups and alcoholic hydroxyl groups in the molecule.

7. A process as claimed in any of claims 1 to 6, wherein the hydrogenation is carried out under neutral or basic reaction conditions.

8. A process as claimed in any of claims 1 to 7, wherein the hydrogenation is carried out from 20 to 250°C.

9. A process as claimed in any of claims 1 to 8, where the nitro-containing reaction products employed are those from polymers of isobutene with an average degree of polymerization of P = 5-100, where up to 50 % by weight of the isobutene can be replaced by other C₂-C₆-olefins as comonomers, with nitrogen oxides or mixtures of nitrogen oxides and oxygen.

10. A process as claimed in any of claims 1 to 8, where the nitro-containing reaction products employed are those from polymers of C₂-C₆-olefins with an average degree of polymerization P = 5-100 and nitrogen oxides or mixtures of nitrogen oxides and oxygen, which are in the form of a mixture of various nitro-containing alkanes and contain as main components the compounds of the general formula I and II 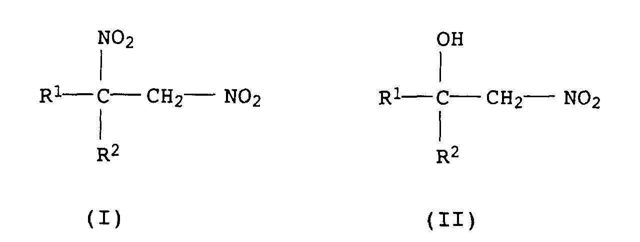 where
R¹ is a long-chain linear or branched alkyl radical with 8-600 carbon atoms and
R² is hydrogen or C₁-C₃-alkyl.

11. A process as claimed in any of claims 1 to 8, where the nitro-containing reaction products employed are those from polymers of C₂-C₆-olefins with an average degree of polymerization P = 5-100 and a high content of β double bonds and a low content of terminal double bonds with nitrogen oxides or mixtures of nitrogen oxides and oxygen, which are in the form of a mixture of various nitro-containing alkanes and contain as main components the compounds of the general formula III and IV 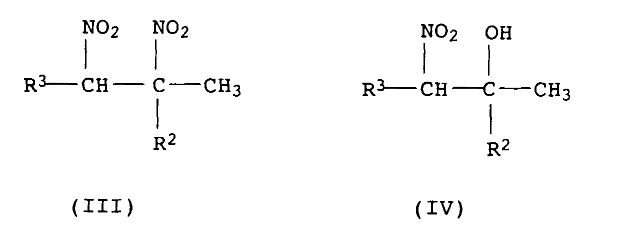 where
R³ is a long-chain linear or branched alkyl radical with 8-600 carbon atoms and
R² is hydrogen or C₁-C₃-alkyl.

12. A process as claimed in any of claims 1 to 8, where the nitro-containing reaction products employed are those from polyisobutenes with an average degree of polymerization P = 10-100 with a content E = 60-90 % of double bonds able to react with maleic anhydride, where E = 100 % would correspond to the calculated theoretical value for the case where each molecule of polyisobutene had such a reactive double bond, with nitrogen oxides or mixtures of nitrogen oxides and oxygen.

13. A process as claimed in any of claims 1 to 8, where the nitro-containing reaction products employed are those from polyisobutenes with an average degree of polymerization P = 10-100 with a content E = 60-90 % of double bonds able to react with maleic anhydride, where E = 100 % would correspond to the calculated theoretical value for the case where each molecule of the polyisobutene had such a reactive double bond, with nitrogen oxides or mixtures of nitrogen oxides and oxygen, which are in the form of a mixture of various nitro-containing alkanes and contain as main components the compounds of the general formula V to VIII

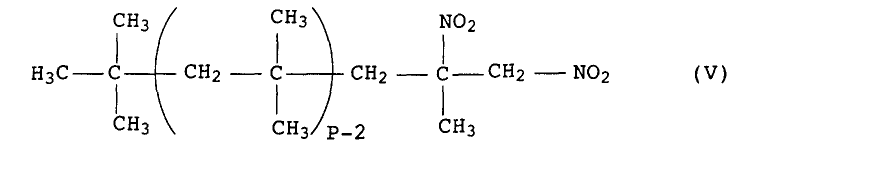

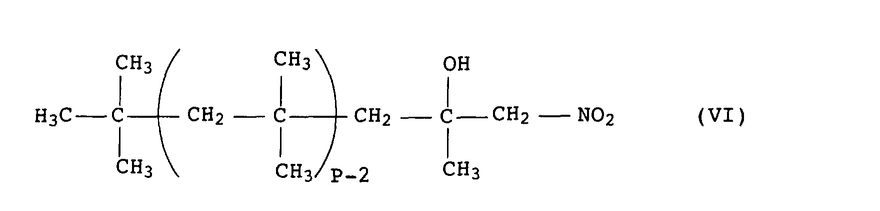

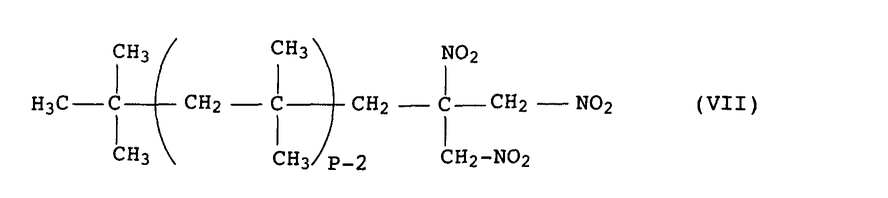

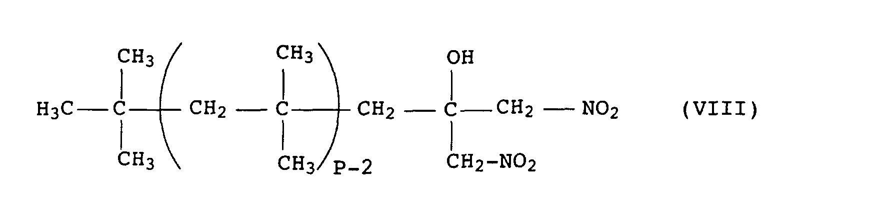

14. A mixture of alkyloximes and alkylnitrones and, where appropriate, aminoalkanes with the same long-chain radicals, which contain as main components the compounds of the general formula XII to XIV 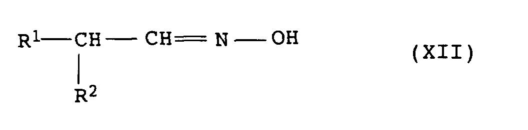 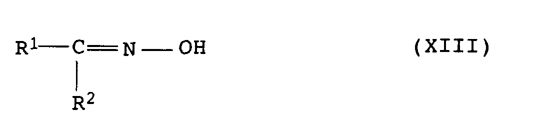 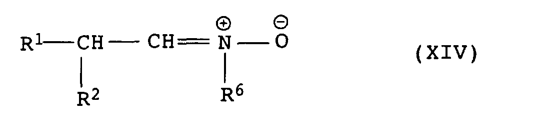 where
R¹ is a long-chain linear or branched alkyl radical with 8-600 carbon atoms,
R² is hydrogen or C₁-C₃-alkyl, and
R⁶ is C₁-C₃-alkyl.

15. A mixture as claimed in claim 14, where the ratio by weight of the three stated main components is (1-98):(1-98):(1-98).

## Revendications

1. Procédé de préparation de composés organiques azotés qui ne portent qu'un groupement fonctionnel azoté et pas de groupes hydroxyle alcoolique dans la molécule, à partir de produits réactionnels, contenant des groupes nitro, entre des polymères d'oléfines en C₂ à C₆, ayant un degré de polymérisation moyen P = 5 à 100, et des oxydes d'azote ou des mélanges d'oxydes d'azote et d'oxygène, caractérisé en ce que l'on fait subir une hydrogénation aux produits réactionnels contenant des groupes nitro, directement à la suite de leur formation en partant de polymères oléfiniques et d'oxydes d'azote.

2. Procédé selon la revendication 1 de préparation d'aminoalcanes, alkyloximes, alkylnitrones ou des mélanges de ceux-ci en tant que composés organiques azotés.

3. Procédé de préparation de composés organiques azotés qui ne portent qu'un groupement fonctionnel azoté et pas de groupes hydroxyle alcoolique dans la molécule, à partir de produits réactionnels, contenant des groupes nitro, entre des polymères d'oléfines en C₂ à C₆, ayant un degré moyen de polymérisation P = 5 à 100, et des oxydes d'azote ou des mélanges d'oxydes d'azote et d'oxygène, caractérisé en ce que l'on transforme les produits réactionnels contenant des groupes nitro, à la suite de leur formation, en des alcènes contenant des groupes nitro par élimination avec des bases et qu'ensuite on fait subir une hydrogénation à ceux-ci, les aminoalcanes résultants étant toujours produits sous forme de mélanges de composés ayant un nombre différent d'atomes de C.

4. Procédé de préparation de composés organiques azotés qui ne portent qu'un groupement fonctionnel azoté et pas de groupes hydroxyle alcoolique dans la molécule, à partir de produits réactionnels, contenant des groupes nitro, entre des polymères d'oléfines en C₂ à C₆, ayant un degré de polymérisation moyen P = 5 à 100, et des oxydes d'azote ou des mélanges d'oxydes d'azote et d'oxygène, caractérisé en ce que l'on transforme les produits réactionnels contenant des groupes nitro en des composés ayant des fonctions carbonyles réactives, on convertit ceux-ci en imines avec de l'ammoniac ou des amines primaires, les composés azotés organiques résultants, provenant des amines primaires mises en oeuvre, pouvant également porter plusieurs groupes fonctionnels azotés et groupes hydroxyle alcoolique dans la molécule, et ensuite on leur fait subir une hydrogénation.

5. Procédé selon les revendications 1 à 4, caractérisé en ce que l'on réalise l'hydrogénation sous forme d'une hydrogénation catalytique avec de l'hydrogène en présence de catalyseurs d'hydrogénation, sous forme d'une hydrogénation par transfert avec des composés moléculaires organiques ou inorganiques ayant un effet réducteur, sous forme d'une réduction avec des métaux communs ou sous forme d'une réduction avec des hydrures complexes du type sel, ou des composés du soufre à faible valence du type sel.

6. Procédé selon les revendications 1 à 5, caractérisé en ce que l'on réalise l'hydrogénation en présence d'ammoniac ou d'amines, de diamines, de polyamines, d'alcanolamines, d'étheramines, de polyétheramines ou de polyétheralcanolamines primaires, secondaires ou tertiaires, les composés organiques azotés résultants provenant des amines et dérivés d'amines mis en oeuvre pouvant porter également plusieurs groupes fonctionnels azotés et groupes hydroxyle alcoolique dans la molécule.

7. Procédé selon les revendications 1 à 6, caractérisé en ce que l'on réalise l'hydrogénation dans des conditions réactionnels neutres ou basiques.

8. Procédé selon les revendications 1 à 7, caractérisé en ce que l'on réalise l'hydrogénation à des températures de 20 à 250°C.

9. Procédé selon les revendications 1 à 8, dans lequel on met en oeuvre en tant que produits réactionnels, contenant des groupes nitro, entre des polymères, parmi ceux de l'isobutène ayant un degré moyen de polymérisation P = 5 à 100, dans lesquels jusqu'à 50% en poids d'isobutène peuvent être remplacés par d'autres oléfines en C₂ à C₆ en tant que comonomères, et des oxydes d'azote ou des mélanges d'oxydes d'azote et d'oxygène.

10. Procédé selon les revendications 1 à 8, dans lequel on met en oeuvre en tant que produits réactionnels, contenant des groupes nitro, entre des polymères, parmi ceux d'oléfines en C₂ à C₆ et ayant un degré moyen de polymérisation P = 5 à 100, et des oxydes d'azote ou des mélanges d'oxydes d'azote et d'oxygène, qui existent sous forme d'un mélange d'alcanes contenant plusieurs groupes nitro, et qui contiennent en tant que composants principaux les composés de formules générales I et II 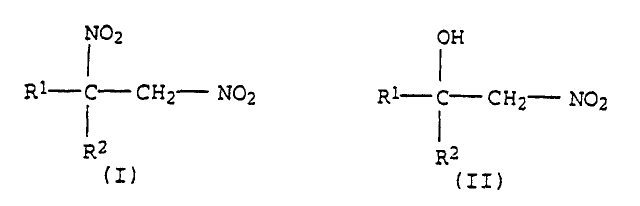 dans lesquelles
R¹ représente un résidu alkyle à longue chaîne, linéaire ou ramifié ayant 8 à 600 atomes de C, et
R² représente un atome d'hydrogène ou un groupe alkyle en C₂ à C₃.

11. Procédé selon les revendications 1 à 8, dans lequel on met en oeuvre en tant que produits réactionnels, contenant des groupes nitro, entre des polymères, parmi ceux d'oléfines en C₂ à C₆, ayant un degré moyen de polymérisation P = 5 à 100, une teneur élevée en doubles liaisons en β et une faible teneur en doubles liaisons terminales, et des oxydes d'azote ou des mélanges d'oxydes d'azote et d'oxygène, qui existent sous forme d'un mélange d'alcanes contenant plusieurs groupes nitro, et contiennent comme composants principaux les composés de formules générales III et IV 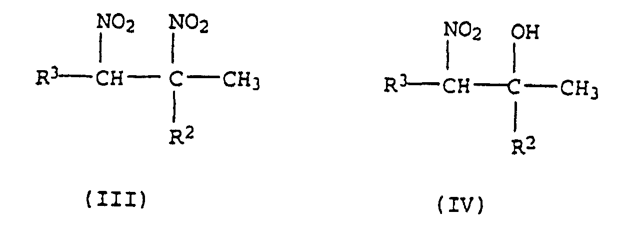 dans lesquelles
R³ représente un résidu alkyle à longue chaîne, linéaire ou ramifié ayant de 8 à 600 atomes de C, et
R² représente un atome d'hydrogène ou un groupe alkyle en C₁ à C₃.

12. Procédé selon les revendications 1 à 8, dans lequel on met en oeuvre en tant que produits réactionnels, contenant des groupes nitro, entre des polyisobutènes, parmi ceux ayant un degré moyen de polymérisation P = 10 à 100 avec une teneur E = 60 à 90% en doubles liaisons qui peuvent réagir avec l'anhydride maléique, E = 100% correspondant à la valeur calculée théorique pour le cas où chaque molécule du polyisobutène aurait une double liaison réactive de ce type, et des oxydes d'azote ou des mélanges d'oxydes d'azote et d'oxygène.

13. Procédé selon les revendications 1 à 8, dans lequel on met en oeuvre en tant que produits réactionnels, contenant des groupes nitro, entre des polyisobutènes, parmi ceux ayant un degré moyen de polymérisation P = 10 à 100, avec une teneur E = 60 à 90% en doubles liaisons qui peuvent réagir avec l'anhydride maléique, E = 100 correspondant à la valeur calculée théorique pour le cas où chaque molécule du polyisobutène aurait une double liaison réactive de ce type, et des oxydes d'azote ou des mélanges d'oxydes d'azote et d'oxygène, qui existent sous forme d'un mélange d'alcanes contenant différents groupes nitro et qui contiennent en tant que composants principaux les composés de formules générales V à VIII

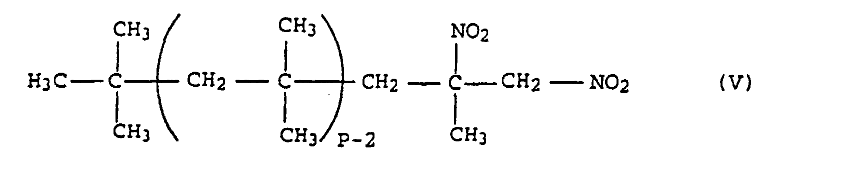

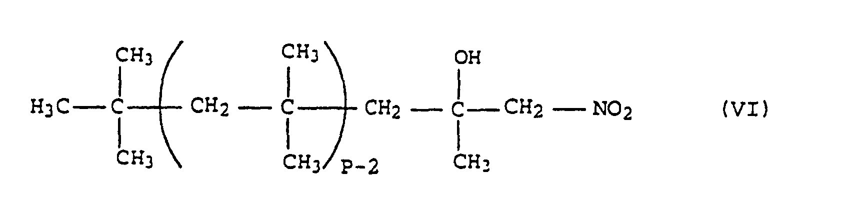

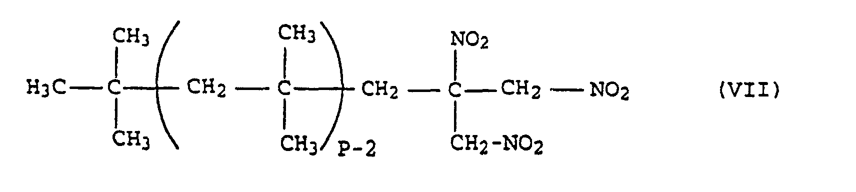

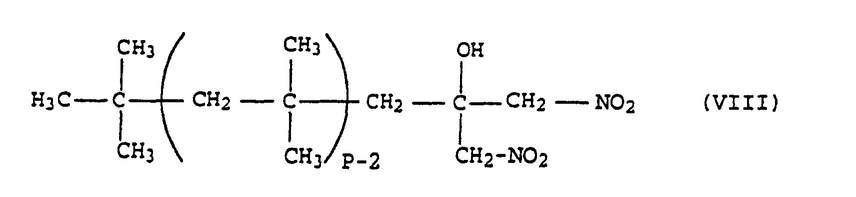

14. Mélanges d'alkyloximes et d'alkylnitrones et éventuellement d'aminoalcanes avec les mêmes résidus à longue chaîne, qui contiennent en tant que composants principaux les composés de formules générales XII à XIV 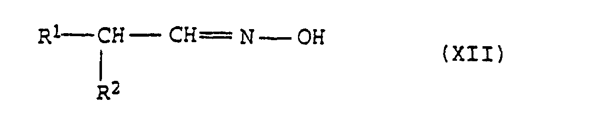 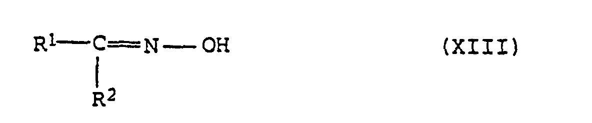 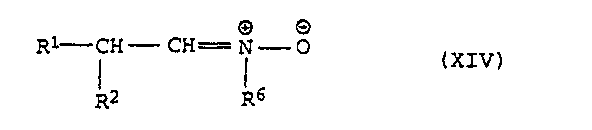 dans lesquelles
R1 représente un résidu alkyle à longue chaîne, linéaire ou ramifié ayant 8 à 600 atomes de C,
R2 représente un atome d'hydrogène ou un groupe alkyle en C₁ à C₃, et
R6 représente un groupe alkyle en C₁ à C₃.

15. Mélanges selon la revendication 14, dans lesquels les trois composants principaux indiqués se trouvent dans un rapport pondéral de (1 à 98):(1 à 98):(1 à 98).
